# NOUVEAU FASCICULE DE BREVET EUROPEEN

(11) **EP 2 526 888 B2**
(45) Date de publication et mention de la décision concernant l'opposition: **27.07.2022**
(45) Mention de la délivrance du brevet: 18.04.2018
(21) Numéro de dépôt: 12177688.4
(22) Date de dépôt: 28.12.2010
(51) Int. Cl.: A61B 17/88, A61B 17/70

(54) **Dispositif pour la chirurgie rachidienne.**
Vorrichtung für die Wirbelchirurgie
Device for spinal surgery

(30) Priorité: 28.12.2009 FR 0906369
(43) Date de publication de la demande: 28.11.2012
(62) Demande divisionnaire de: 10810769.9
(73) Titulaire: Safe Orthopaedics, 95610 Eragny-sur-Oise (FR)
(72) Inventeur: Petit, Dominique, 62180 Verton (FR)
(74) Mandataire: IP Trust

(56) Documents cités:
- WO-A2-2007/092870
- WO-A2-2008/097974
- US-A1- 2004 138 662
- US-A1- 2005 192 570
- US-A1- 2006 217 719
- US-A1- 2008 262 318
- US-A1- 2009 222 045
- US-A1- 2009 222 045

## Description

L'invention concerne un dispositif permettant de réaliser une stabilisation rachidienne par élément d'ancrage osseux de type vis par voies postérieure ou postéro-latérale.

Le dispositif selon l'invention est destiné notamment, mais non exclusivement, à la chirurgie d'ostéosynthèse rachidienne lombaire, thoracique, ou encore cervicale postérieure par voie chirurgicale minimalement invasive.

De manière classique en soi, il est procédé lors de dysfonctionnements anatomiques de la colonne vertébrale à la mise en place d'ancrages osseux de type vis pédiculaire dans les vertèbres reliées entre elles par des éléments de liaison de type tiges ou plaques.

L'état de l'art dans le domaine de la chirurgie de la colonne vertébrale consiste à mettre à disposition des hôpitaux des implants, et des sets d'instruments pour la pose de ceux-ci. Certaines sociétés spécialisées du domaine délivrent des implants conditionnés stériles prêts à l'emploi. Les sets d'instruments réutilisables après décontamination et stérilisation comportent de nombreux désavantages. Le risque de contamination d'un patient à un autre est très élevé; le nettoyage, la décontamination et stérilisation sont des étapes parfois quasi impossibles à réaliser correctement à la vue des designs complexes, avec de nombreuses cavités dans les instruments. Ces étapes sont à la charge financière des hôpitaux et représentent des coûts très importants à la fois humains et matériels.

La perte, la casse, l'usure et la détérioration d'un instrument peuvent entrainer des conséquences désastreuses pour le patient, voir engendrer l'annulation de la chirurgie. La logistique comporte également de nombreux désavantages, lourde et coûteuse à la fois pour l'industriel et l'hôpital, elle implique le plus souvent des inventaires importants. En effet, les chirurgiens n'opérant dans la majorité des cas que deux jours par semaine, le nombre de sets d'instruments mis à disposition de l'hôpital est fonction de l'activité, plus le cas échéant, des chirurgies de traumatologie venant s'ajouter au programme opératoire. Voir également la demande US 2004/0138662.

Les rotations de sets entre l'hôpital et le service logistique de l'industriel sont donc très nombreuses, ce qui augmente significativement les risques de pertes et d'erreurs.

Dans d'autres domaines chirurgicaux, pour prendre en compte l'aspect sécurité à la fois pour le patient et le chirurgien, l'aspect financier humain et matériel pour l'hôpital ainsi que le service offert et attendu par la clientèle de l'industriel, il a été proposé de s'orienter vers une chirurgie en « tout stérile » à usage unique. On connait par exemple la demande internationale WO 98/22035. Il y est décrit une trousse d'instruments chirurgicaux, fabriquée de façon économique en matière synthétique et stérile. Ce type de trousse est commercialisé par exemple pour des actes de génécologie ou micro chirurgie. Ces instruments génériques de type pince ciseaux, de dissection ou encore scalpel n'ont pas pour fonction la pose d'un implant orthopédique, et ne sont pas amenés à subir des contraintes mécaniques élevées. La transposition d'un instrument métallique en polymère à usage unique est connue dans de nombreux domaines médicaux.

On connait également la demande internationale WO 2005/016183. Il y est décrit un kit d'implants de type plaque et vis vertébrales associé à des instruments conditionnés stérile à usage unique. Hormis le désavantage de ne proposer qu'un seul diamètre et longueur de vis pour l'ancrage osseux, ce procédé ne décrit qu'un seul type d'instrument, une barre de tournevis et sa poignée. Cette demande internationale ne propose pas de solution technique pour la réalisation et fabrication des instruments soumis à de fortes contraintes comme le distracteur de vertèbres absolument indispensable à la pose de l'implant.

La présente invention entend répondre dans sa globalité aux problèmes liés à l'utilisation de matériaux polymères pour la fabrication des instruments chirurgicaux à usage unique conditionnés stérile, et plus particulièrement pour la chirurgie rachidienne de type vis pédiculaire.

A cet effet, et selon un premier aspect, l'invention propose un élément d'ancrage osseux de type vis pédiculaire pré-monté avec un tube de montage multifonction, le tout préparé en usine dans un emballage scellé de conditionnement stérile à usage unique. Plus spécifiquement, elle a trait à un dispositif rachidien pour la fixation des vertèbres par voie postérieure ou postéro-latérale selon la revendication 1.

Suivant un mode de réalisation, le tube de montage comporte une ouverture longitudinale débouchant en partie proximale de l'élément d'ancrage osseux pour le passage de l'élément de liaison.

Avantageusement les tubes pré-montés sont démontables après implantation de l'élément d'ancrage osseux et de son élément de blocage .

Suivant un mode de réalisation, le diamètre intérieur du tube de montage permet le passage d'un élément de blocage de l'élément de liaison sur l'élément d'ancrage osseux.

Suivant un mode de réalisation, le diamètre intérieur du tube de montage permet le passage d'ancillaires pour la mise en place d'un élément de blocage.

.

Avantageusement, le tube de montage est pourvu de moyens permettant de maintenir l'ancillaire dans l'alignement de l'élément d'ancrage osseux. Selon une configuration particulière, les moyens de maintien comprennent des ergots ménagés sur la face interne du tube de montage. Il peut être prévu, en complément ou en remplacement des ergots, une ailette de maintien montée sur l'extrémité distale du tube de montage de manière à maintenir l'ancillaire dans une position donnée par rapport au tube de montage et à l'élément d'ancrage osseux.

Selon une variante particulière, le tube de montage sont pourvu d'au moins une queue d'aronde dans son passage intérieure complémentaire à l'ancillaire pour la mise en place de l'élément de blocage afin d'obtenir une interconnexion suffisamment résistante au regard des contraintes exercées sur cet ensemble.

Suivant un mode de réalisation, le tube de montage est réalisé en matière composite, polymère, alliage ferreux ou non ferreux ou encore une combinaison de ses différentes matières.

Suivant un mode de réalisation, le tube de montage est surmoulé sur l'élément d'ancrage osseux.

Suivant un mode de réalisation, le tube de montage est réalisé en un ou plusieurs matériaux, l'un au moins desdits matériaux étant incompatible avec un cycle de stérilisation à l'autoclave comportant une phase de maintien à 134°C pendant 18 minutes.

Suivant un mode de réalisation, le tube de montage comporte en extrémité distale une empreinte pour la mise en place d'un élément de maintien perpendiculaire de type poignée. Cet élément perpendiculaire de maintien est utilisé principalement lors du serrage de l'élément de blocage avec un effet de contre couple, évitant ainsi la rotation de l'ensemble de l'élément d'ancrage osseux et tube de montage.

Suivant un mode de réalisation, le tube de montage comporte en extrémité distale une zone filetée ou taraudée. Ce filetage ou taraudage est prévu pour recevoir un élément de poussée du tube porte élément de blocage décrit ci dessous.

Suivant un mode de réalisation, le tube de montage est composé de deux demi-coquilles.

Suivant un mode de réalisation, les deux demi-coquilles sont identiques.

Suivant un mode de réalisation, les deux demi-coquilles sont jointives dans leurs parties distales.

Suivant un mode de réalisation, les deux demi-coquilles comportent dans leurs parties jointives un moyen de tenon mortaise de positionnement, ou moyens similaires d'indexation afin de garantir le bon positionnement de ces deux demi-coquilles.

Suivant un mode de réalisation, les deux demi-coquilles sont collées, soudées, ou encore clipsées.

Selon une variante particulière, la connexion proximale de la demi-coquille sur l'élément d'ancrage osseux est assurée par une empreinte complémentaire à la forme de cette demi-coquille de telle façon que l'assemblage ou le démontage se fasse par rotation de la demi-coquille autour de l'élément d'ancrage osseux.

Selon cette variante particulière, une fois les deux demi-coquilles interconnectées sur leurs parties distales, il est impossible de les désolidariser de l'élément d'ancrage osseux, tout en permettant un démontage facile que les deux demi-coquilles sont déconnectées.

Suivant un mode de réalisation, les deux demi-coquilles sont maintenus solidairement, de préférence par l'intermédiaire d'une bague en extrémité distale de ces dites demi-coquilles.

Avantageusement et selon cette variante particulière la partie distale de cette bague est pourvue d'un filetage, et d'une empreinte. Ce filetage est prévu pour recevoir un élément de poussée du tube porte élément de blocage, et l'empreinte est prévue pour recevoir un élément perpendiculaire de maintien.

Suivant un mode de réalisation, la bague comporte au moins une forme de type baïonnette venant s'interconnecter sur des pions aménagés sur les deux demi-coquilles, ce qui permet un montage simple et rapide de cette dite bague.

Suivant un mode de réalisation, un ressort de compression se situe entre la bague et les deux demi-coquilles afin de garantir un maintien sécurisé de l'ensemble.

Suivant un mode de réalisation, la bague comporte au moins un axe traversant entre la bague et les deux demi-coquilles.

Suivant un mode de réalisation, le dimensionnement de l'axe est défini pour rompre sous un effort de cisaillement suivant un couple donné.

Avantageusement, le tube de montage et la bague sont agencés pour former un ensemble rigide.

Avantageusement, les deux demi-coquilles sont agencées pour former, lorsqu'elles sont maintenues solidairement l'une à l'autre, un tube de guidage.

Selon l'invention, les deux demi-coquilles présentent chacune une extrémité proximale agencée pour venir en prise avec l'élément d'ancrage osseux.

Avantageusement et selon cette variante particulière, ce couple de rupture est identique au couple de serrage de l'élément de blocage qui solidarise l'élément de liaison tige dans l'élément d'ancrage osseux.

Avantageusement et selon cette variante particulière cette rupture conditionne le démontage de la bague, donc également des deux demi-coquilles.

Suivant un mode de réalisation, l'élément de blocage est pré-monté solidairement avec un tube porte élément de blocage pour former un ensemble de blocage, l'élément de blocage étant démontable du tube porte élément de blocage.

Avantageusement, ce maintien est assuré par un taraudage.

Selon une variante particulière, ce taraudage peut être légèrement différent du filetage de l'élément de blocage afin d'assurer son maintien par coincement.

Selon une variante particulière, le maintien de l'élément de blocage est assuré par un clipsage.

Avantageusement, l'ensemble de blocage peut être conditionné stérile.

Suivant un mode de réalisation, l'ensemble de blocage est conditionné stérile dans l'emballage scellé de conditionnement.

Suivant un mode de réalisation, le tube porte élément de blocage présente des dimensions permettant son insertion dans le tube de montage. Le tube de montage peut avantageusement présenter une paroi intérieure tubulaire pourvue d'au moins un relief d'indexation, le tube porte élément de blocage présentant un relief d'indexation complémentaire de forme complémentaire au relief d'indexation du tube de montage. Le tube porte élément de blocage peut par ailleurs présenter une partie proximale pourvue d'un relief d'appui, situé en regard de l'interface de solidarisation de l'élément d'ancrage osseux lorsque le tube porte élément de blocage est inséré dans le tube de montage, pour appuyer un élément de liaison sur l'interface de solidarisation de l'élément d'ancrage osseux. Le dispositif peut comporter en outre un élément de poussée coopérant avec le tube porte élément de blocage pour insérer le tube porte élément de blocage dans le tube de montage.

Avantageusement, l'extrémité proximale est pourvue de deux pattes de part et d'autre de l'élément de blocage, d'une longueur au moins équivalente à la hauteur de l'élément de blocage par rapport à la face de celui-ci devant venir en contact de la tige. La largeur de ces pattes ne peut être supérieure au diamètre de l'élément de liaison tige. La fonction de ces deux pattes est de permettre l'appui sur l'élément de liaison tige sans contact de l'élément de blocage ni sur l'élément d'ancrage osseux, ni sur l'élément de liaison tige.

Selon une variante particulière, la forme extérieure du tube porte élément de blocage est pourvue de deux queues d'aronde de forme complémentaire aux deux demi-coquilles.

Ces queues d'aronde complémentaires entre les deux demi-coquilles et le tube porte élément de blocage renforcent considérablement l'ensemble et évitent ainsi tout risque d'écartement sous contraintes élevées, des deux demi-coquilles et donne une résistance mécanique supérieure en flexion et torsion.

Selon une variante particulière, la longueur totale du tube porte élément de blocage, est supérieure aux deux demi-coquilles, ou bien aux deux demi-coquilles et à la bague.

Selon cette variante particulière, la partie distale du tube porte élément de blocage et la forme intérieure de l'élément de poussée sont compatibles avec la mise en place de l'élément de liaison tige au fond du logement en forme de U prévue de l'élément d'ancrage osseux. Le tube porte élément de blocage fait donc office également de pousse tige.

Suivant un mode de réalisation, l'élément de poussée comprend un filetage ou un taraudage coopérant avec la zone filetée ou taraudée du tube de montage, pour la mise en place de l'élément de liaison.

Avantageusement, le tube de montage, lorsque le tube porte élément de blocage est positionné en son sein, forme un tube de guidage.

Avantageusement, le tube de montage et le tube porte élément de blocage, lorsqu'il est positionné en son sein, forment un ensemble rigide.

Suivant un mode de réalisation, le tube porte élément de blocage présente une forme intérieure permettant le passage d'un tournevis, pour le serrage ou le desserrage de l'élément de blocage.

Suivant un mode de réalisation, le tube porte élément de blocage est réalisé en matière composite, polymère, alliage ferreux ou non ferreux ou encore une combinaison de ses différentes matières.

Suivant un mode de réalisation, toutes les pièces du dispositif sont à usage unique.

Suivant un mode de réalisation, toutes les pièces du dispositif sont conditionnées de façon stérile dans un ou plusieurs emballages scellés.

Dans ce qui précède, l'accent a été mis sur l'existence d'un emballage stérile pour l'élément d'ancrage osseux et le tube de montage pré-monté. Toutefois, suivant un autre aspect de l'invention, celle-ci couvre également les caractéristiques mécaniques du tube et de sa fixation à l'élément d'ancrage osseux, indépendamment de leur conditionnement stérile. Ainsi, l'invention a également trait à un dispositif rachidien pour la fixation des vertèbres par voie postérieure ou postéro-latérale, comportant un élément d'ancrage et un tube, et se distinguant par une ou plusieurs des caractéristiques décrites aux paragraphes [0011] à [0051], prises individuellement ou en combinaison.

Selon un autre aspect de l'invention, celle-ci a trait à un kit d'instruments pour la pose ou la dépose d'un implant rachidien comportant au moins deux éléments d'ancrage osseux filetés, un organe de liaison de type tige ou plaque reliant mécaniquement les éléments d'ancrage osseux et des éléments de blocage pour verrouiller en position l'élément de liaison par rapport aux éléments d'ancrage, pour de réaliser la totalité des gestes chirurgicaux liés à la pose ou la dépose dudit implant, caractérisé en ce que la totalité de ces instruments nécessaires sont à usage unique, conditionnés stérile dans un ou plusieurs emballages scellés.

Selon une variante particulière, le kit d'instruments est réduit pour la pose ou la dépose des implants. Il est constitué en principal de deux barres de tournevis (élément d'ancrage osseux et élément de blocage), de deux poignées multifonctions, d'une pince porte tige, et d'une pince de compression distraction, soit six éléments au total.

Avantageusement, le kit d'instrumentation pour la pose ou la dépose des implants n'est composé que de six instruments grâce à leurs designs multifonctions et aux parties tubes de montage et porte élément de blocage pré-montés sur ces dits implants.

Avantageusement, et selon une variante particulière, l'invention propose le contrôle du couple de serrage de l'élément de blocage afin de sécuriser le maintien de l'élément de liaison tige sur les éléments d'ancrage osseux de types vis pédiculaires.

Avantageusement, l'invention permet au chirurgien l'approche chirurgicale, minimale invasive.

Selon un mode de réalisation, les instruments sont réalisés en matière composite, polymère, alliage ferreux ou non ferreux ou encore une combinaison de ses différentes matières.

Suivant un mode de réalisation, le dispositif d'instruments à usage unique comprend au moins une barre tournevis, une poignée et une pince.

Suivant un mode de réalisation, le kit comporte une poignée en «T» équipée d'un limiteur de couple pour le serrage final dynamométrique de l'élément de blocage. Le couple de serrage de l'élément de blocage est optimum pour garantir le maintien de l'élément de liaison tige dans l'élément d'ancrage osseux.

Avantageusement l'élément de poussée du tube porte élément de blocage / introducteur de tige est également la poignée en T qui est pourvue d'un filetage correspondant à celui de l'extrémité distale des deux demi-coquilles ou de la bague.

Avantageusement cette poignée en T est prévue pour recevoir deux barres de tournevis, pour l'élément d'ancrage osseux et l'élément de blocage.

Avantageusement cette poignée en T est canulée sur toute sa longueur laissant passer au travers les barres de tournevis.

Selon cette configuration particulière, les parties proximales d'engrènement des barres de tournevis vis pédiculaire et bouchon sont différentes.

Selon cette configuration particulière, la fonction dynamométrique de cette poignée en T n'est applicable qu'à la barre de tournevis de serrage de l'élément de blocage.

Suivant un mode de réalisation, le kit comporte une poignée droite permettant la mise en place ou le démontage des éléments d'ancrage osseux, le serrage ou le desserrage provisoire des éléments de blocage, et le maintien perpendiculaire du dispositif.

Avantageusement cette poignée droite est prévue pour recevoir les différentes barres de tournevis (vis pédiculaire et bouchon).

Avantageusement cette poignée droite est canulée sur toute sa longueur laissant passer au travers les différentes barres de tournevis.

Avantageusement, on peut prévoir que la poignée droite permette également une utilisation en T pour la mise en place ou le démontage des éléments d'ancrage osseux et le serrage ou le desserrage des éléments de blocage, de telle sorte qu'un couple plus important soit plus facile à appliquer.

Avantageusement, l'élément perpendiculaire de maintien est également la poignée droite. Celle-ci est pourvue sur l'une de ses extrémités d'une empreinte complémentaire à celle de l'extrémité distale des deux demi-coquilles ou de la bague.

Suivant un mode de réalisation, la poignée droite peut être agencée pour permettre le maintien de l'élément d'ancrage osseux pour le serrage ou le desserrage des éléments de blocage. Cette poignée droite est pourvue à l'une des ses extrémités d'une forme complémentaire à la tête de l'élément d'ancrage osseux.

Avantageusement, cette poignée droite permet donc lors du serrage ou desserrage de l'élément de blocage d'effectuer un effet contre couple aussi bien quand les deux demi-coquilles ou demi-coquilles + bague sont en place que lorsque celles-ci sont démontées.

Suivant un mode de réalisation, la poignée droite peut également permettre la préhension de l'élément de blocage et son guidage sur l'élément d'ancrage osseux. Cette préhension est agencée pour maintenir de façon sécurisée l'élément de blocage dans son extrémité proche de la forme complémentaire à la tête de l'implant d'ancrage osseux.

Avantageusement, ce maintien est assuré par un taraudage.

Selon une variante particulière, ce taraudage peut être légèrement différent du filetage de l'élément de blocage afin d'assurer son maintien par coincement.

Selon une variante particulière, le maintien de l'élément de blocage est assuré par un clipsage.

Avantageusement, le kit d'instrument comporte une pince de compression et une pince de distraction des éléments d'ancrage osseux, les pinces de compression et de distraction ne formant qu'une seule pince.

Suivant un mode de réalisation, la pince de manœuvre comprend deux bras permettant la compression et la distraction des éléments d'ancrage osseux. Cette pince de manœuvre à deux bras peut comporter des extrémités proximales courbes avec un crantage sur les parties convexes.

Avantageusement, cette pince de manœuvre à deux bras comporte des extrémités proximales courbes avec une indexation longitudinale. L'indexation peut être réalisée par une rainure sur l'un des bras, et un pion saillant sur l'autre bras.

Avantageusement, le kit d'instruments peut comprendre une pince (900) comportant deux bras croisés symétriques par rapport à un plan de référence et fixés l'un à l'autre par une articulation, chaque bras présentant une extrémité en forme de fourche pour chevaucher une tige, les extrémités en forme de fourche de chaque bras étant disposées en vis-à-vis l'une de l'autre par rapport au plan de symétrie, les extrémités des bras formant des extrémités de compression, les extrémités opposées aux extrémités de compression de chaque bras étant agencées pour permettre la préhension de la tige lors d'un mouvement de rapprochement des extrémités de compression l'une vers l'autre.

Le kit d'instruments peut également comprendre une pince comportant deux bras symétriques par rapport à un plan de référence et joints l'un à l'autre par une articulation, chaque bras présentant des première et deuxième extrémités en forme de fourche pour chevaucher une tige, l'une des extrémités d'un des bras étant agencée avec l'extrémité de l'autre bras disposée en vis-à-vis d'elle par rapport au plan de symétrie pour former des extrémités de distraction, les autres extrémités formant des extrémités de compression.

Suivant un autre aspect de l'invention, celle-ci a trait à une opération de pose ou de dépose d'un implant rachidien comportant au moins deux éléments d'ancrage osseux filetés, un organe de liaison de type tige ou plaque reliant mécaniquement les éléments d'ancrage osseux et des éléments de blocage pour verrouiller en position l'élément de liaison par rapport aux éléments d'ancrage, caractérisée en ce que les instruments coopérant avec les éléments d'ancrage osseux et les éléments de blocage lors de la pose ou la dépose de l'implant sont sortis d'un ou plusieurs emballages stériles scellés au début de l'opération et sont jetés à la fin de l'opération, sans être re-stérilisés.

D'autres objets et avantages de l'invention apparaîtront au cours de la description qui suit, faite en référence aux dessins annexés, dans lesquels :
- la figure 1 représente une vue schématique d'un dispositif selon un premier mode de réalisation de l'invention ;
- la figure 2 représente une vue schématique du kit d'instruments selon un premier exemple de réalisation ;
- les figures 3a et 3b représentent la connexion entre l'élément d'ancrage osseux et le tube de montage multifonction ;la figure 3c représente un détail de la connexion entre l'élément d'ancrage osseux et le tube de montage multifonction illustrée sur la figure 3a ;
- la figure 3d représente un détail d'une connexion entre l'élément d'ancrage osseux et le tube de montage multifonction selon une variante de réalisation ;
- les figures 4a et 4b représentent différents assemblages de la bague sur les deux demi-coquilles formant le tube de montage multifonction ;
- la figure 5 représente un assemblage particulier des deux demi-coquilles formant le tube de montage multifonction ;
- la figure 6 représente le tube porte élément de blocage ;
- la figure 7 représente le tube porte élément de blocage inséré dans les deux demi-coquilles formant le tube de montage multifonction ;
- la figure 8 représente une coupe de la poignée en T multifonction dans sa version dynamométrique ;
- la figure 9 représente une coupe de la poignée droite multifonction ;
- les figures 10a à 10g représentent les différentes étapes opératoires de mise en place de l'élément d'ancrage d'osseux, de l'élément de liaison tige et de l'élément de blocage ;
- la figure 11 représente une fonction particulière des poignées lors du serrage ou desserrage de l'élément de blocage quand le tube de montage n'est plus en place sur l'implant ;
- la figure 12 représente une vue schématique de la pince de compression/distraction des éléments d'ancrage osseux en version compression ;
- la figure 13 représente une vue schématique de la pince de compression/distraction des éléments d'ancrage osseux en version distraction ;
- la figure 14 représente un moyen d'articulation particulier de cette pince en version distraction ;
- la figure 15 représente une vue schématique d'un dispositif selon un deuxième mode de réalisation de l'invention ;
- la figure 16 représente une vue schématique d'un kit d'instruments selon un deuxième exemple de réalisation de l'invention ;
- la figure 17 représente une vue schématique du tube de montage et de la bague formant le dispositif de la figure 15, la bague étant représentée non montée sur le tube de montage ;
- la figure 18 représente une vue de dessous de la bague de la figure 17 ;
- la figure 19 représente une vue en coupe selon l'axe XIX-XIX du tube de montage de la figure 17 ;
- la figure 20 représente une vue en coupe selon l'axe XX-XX de l'ensemble tube/tournevis de la figure 15 sur lequel l'élément d'ancrage osseux est pré-monté ;
- les figures 21a et 21b représentent respectivement une vue de détail de l'extrémité proximale et de l'extrémité distale du tube illustré sur la figure 20.
- la figure 22 représente une vue schématique d'un ensemble tube/tournevis/poignée selon une variante d'utilisation ;
- les figures 23a et 23b représentent une pince de compression contenue dans le kit d'instruments de la figure 16, la pince de compression étant représentée suivant deux situations d'utilisation ;
- les figures 24a et 24b représentent une pince de distraction contenue dans le kit d'instruments de la figure 16, la pince de distraction étant représentée suivant deux situations d'utilisation ; et
- les figures 25a à 25c représentent les étapes de mise en place de l'élément de blocage lorsque le tube de montage a été démonté.

Pour plus de clarté, les éléments identiques ou similaires des différents modes de réalisation sont repérés par des signes de référence identiques sur l'ensemble des figures.

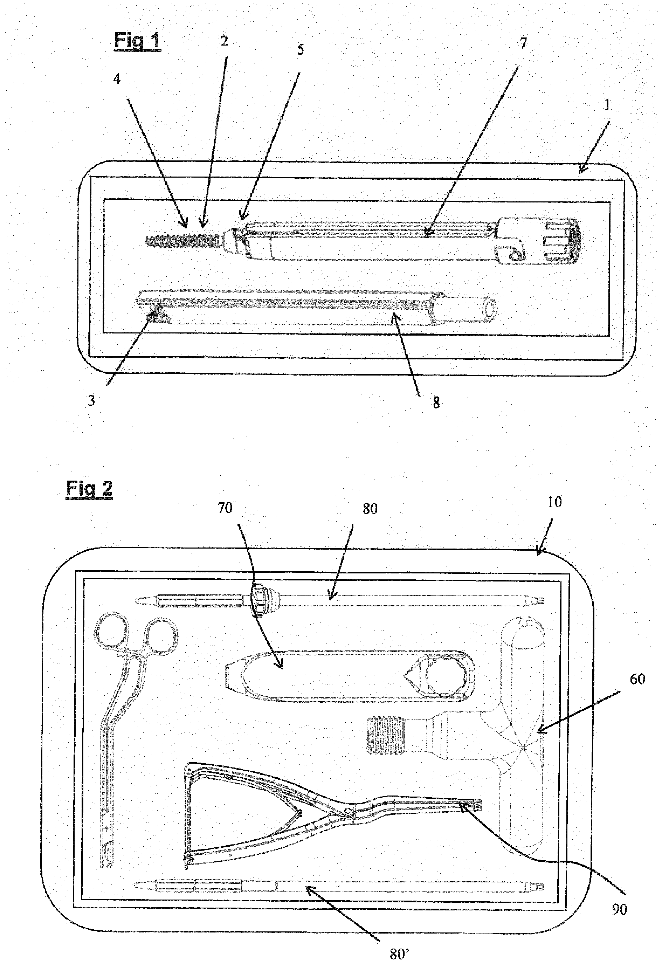

En relation avec la figure 1, il est décrit un dispositif (1) destiné à être fixé sur une vertèbre, comprenant un moyen d'ancrage osseux (2) et son moyen de fermeture (3) respectivement pré-montés solidairement sur des tubes (7) et (8), le tout préparé en usine dans un emballage scellé de conditionnement stérile à usage unique.

Afin de faciliter la lecture de ce qui suit, le dispositif 1 décrit ci dessus sera par la suite appelé « dispositif vis (1) ».

Dans une configuration particulière non représentée, le dispositif vis (1) peut comprendre plusieurs moyens d'ancrage osseux (2), et plusieurs moyens de fermeture (3) pré-montés solidairement sur des tubes (7) et (8) à usage unique, le tout dans un même emballage scellé de conditionnement stérile.

Le moyen d'ancrage osseux (2) est composé d'une partie filetée (4) destinée à être insérer dans l'os, et une partie tête (5) destinée à recevoir un élément de liaison de type tige ou plaque (6).

Afin de faciliter la lecture de ce qui suit l'élément d'ancrage osseux (2) sera par la suite appelé « vis (2) », la partie tête (5) sera par la suite appelée « tête de la vis (5) », l'élément de liaison de type tige ou plaque (6) sera par la suite appelé « tige (6) », et l'élément de blocage (3) sera par la suite appelé « bouchon (3) ». L'ensemble vis (2), tige (6), et bouchon (3) sera par la suite appelé « implant ».

Dans une configuration particulière, la tête de la vis (5) est aménagée avec une forme en U (25) pourvu d'un canal ménagé pour recevoir la tige (6). Avantageusement, le canal est délimité par deux branches pourvue d'un taraudage intérieur (26) destiné à recevoir le bouchon (3) afin de solidariser la vis (2) et la tige (6). La partie filetée (4) de la vis (2) peut être fixe ou bien mobile vis-à-vis de la tête de la vis (5) ; Ce type de vis (2) à bouchon (3) appartient au domaine public et constitue un des états de l'art de la chirurgie rachidienne pour la fixation des vertèbres.

Le matériau le plus fréquemment utilisé pour la fabrication des implants est le titane. Dans une configuration particulière de l'invention le matériau utilisé pour la fabrication peut être tous matériaux implantables connus ou non à ce jour, comme le Peek, l'inox, le chrome cobalt, ou encore un composite à base de fibre de verre ou de carbone. Des revêtements de type HATCP (HydroxyApatite TriCalcium Phosphate) ou autres peuvent également être appliqués pour améliorer l'ancrage osseux ou bien la tenue mécanique globale de l'implant.

Les tubes (7) et (8) à usage unique peuvent être fabriqués dans tout matériaux connus ou non à ce jour comme les composites, les polymères, les métaux ferreux, et non ferreux (aluminium) tant que ceux-ci correspondent aux critères de biocompatibilité liés à l'application. De préférence, le matériau utilisé sera recyclable afin de répondre aux exigences de protection de l'environnement. Des revêtements peuvent également être appliqués afin de répondre aux critères de biocompatibilité ou bien accroitre les caractéristiques mécaniques.

De préférence au moins un au moins desdits matériaux constituant le dispositif (1) est incompatible avec un cycle de stérilisation à l'autoclave comportant une phase de maintien à 134°C pendant 18 minutes.

Le mode de stérilisation choisi sera compatible suivant les caractéristiques desdits matériaux suivant la technique de l'art. Cette stérilisation se fera de préférence suivant une irradiation Gamma ou encore suivant un procédé spécifique à l'oxyde d'éthylène (ETO).

La figure 2 décrit également un kit d'instruments (10) à usage unique, conditionné stérile pour la pose ou la dépose des implants. Au nombre de six instruments dans cette configuration particulière, ils permettent de réaliser la totalité des gestes chirurgicaux exigés pour la pose ou la dépose des implants. Avantageusement les matériaux ou revêtements utilisés pour la fabrication de ce kit d'instruments (10) sont les mêmes que ceux qui peuvent être utilisés pour la fabrication des tubes (7) et (8).

Ce dispositif kit d'instruments (10) comporte beaucoup d'avantages comme celui de réduire le coût global de la chirurgie du rachis, et de garantir la non contamination d'un patient à un autre, et donc de réduire significativement le nombre d'infection nosocomiale.

Ce dispositif kit d'instruments (10) est composé en principal de barres tournevis, de poignées et de pinces.

Avantageusement de façon non limitative ni restrictive, le kit d'instruments (10) permet d'effectuer les gestes chirurgicaux suivants: la mise en place des vis (2) dans les pédicules des vertèbres, le cintrage de la tige (6) pour être en conformité avec l'anatomie du patient, la mise en place de la tige (6) quelques soient les efforts d'introduction, la mise en place du bouchon (3), les manœuvres de correction des vertèbres instrumentées de type compression et distraction, le serrage final contrôlé et sécurisé du bouchon (3).

Avantageusement, et selon une configuration particulière, le kit d'instruments (10) inclut des tiges d'essais permettant au chirurgien de déterminer la longueur et la courbure optimum avant le choix des tiges (6) qui seront implantées.

Avantageusement, le même kit d'instruments (10) peut être utilisé pour la dépose du matériel après ostéosynthèse, ou pour toutes autres raisons cliniques.

Avantageusement, de nombreux instruments constituant ce kit (10), sont multifonctions. L'ensemble des caractéristiques sera détaillé par la suite.

Avantageusement les tubes (7) et (8) pré-montés respectivement sur la vis (2) et le bouchon (3) sont également multifonctions.

Avantageusement, l'association kit d'instruments (10) et tubes (7) et (8) pré-montés, permet au chirurgien l'approche chirurgicaleen approche minimale invasive médiane, latérale ou bilatérale.

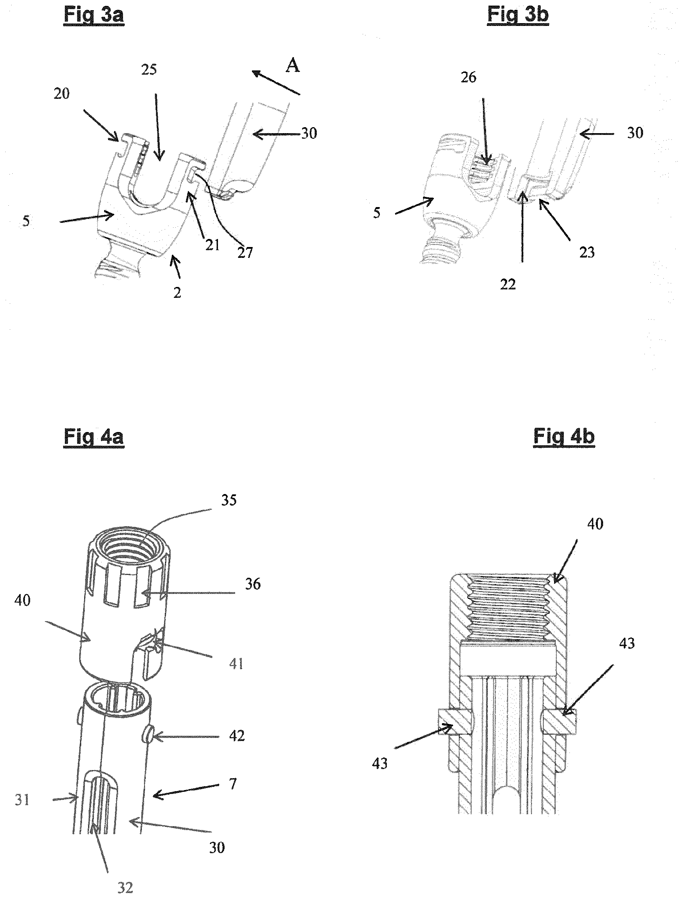

Les figures 3a et 3b décrivent dans le mode de réalisation préféré d'accroche du tube de montage (7) sur la vis (2).

La tête de la vis (5) comporte sur sa surface extérieure deux empreintes (20) ménagées de part et d'autre du canal en U (25). L'empreinte est composée d'un canal (27) et d'un logement intérieur (21). Dans cette configuration, le tube de montage (7) est composé de deux demi-coquilles (30, 31). La forme femelle de l'empreinte sur la tête de vis (5) est reproduite en male sur les extrémités proximales des demi-coquilles (30 et 31). Avantageusement la forme extérieure male de la tête de vis (5) est reproduite en forme intérieure femelle (22) à l'extrémité proximale des deux demi-coquilles afin de solidifier et solidariser dans tout les sens (sauf celui de la rotation avec comme point de centre de rotation l'empreinte intérieure (21)) les deux demi-coquilles (30 et 31) et la vis (2).

Plus particulièrement, chaque demi-coquille présente en extrémité proximale une cavité de forme complémentaire à la forme d'une des branches de la tête de vis (5). Ainsi, lorsque les deux demi coquilles (30, 31) sont montées sur la tête de vis (5), les branches de cette dernière sont emprisonnées dans les cavités ménagées sur chacune des extrémités proximales des demi-coquilles, la paroi délimitant chaque cavité « épousant » sensiblement la surface extérieure des branches de la tête de vis (5). Avantageusement, la cavité de chaque extrémité proximale comporte un ergot ménagé de sorte que, lorsque les extrémités proximales sont placées sur la tête de vis (5), l'ergot vient se loger dans l'empreinte ménagée sur la tête de vis. Dans le mode de réalisation illustré, les évidements sont ménagés au niveau de l'extrémité haute de la tête de vis (5).

Il est bien entendu évident que l'invention ne se limite pas, en ce qui concerne les moyens de maintien de l'implant au tube de montage, aux ergots tels qu'illustrés sur les figures 4 à 6, toutes autres formes d'ergots ou tout autre moyen de maintien d'implant pouvant être prévus sans pour autant sortir du cadre de l'invention.

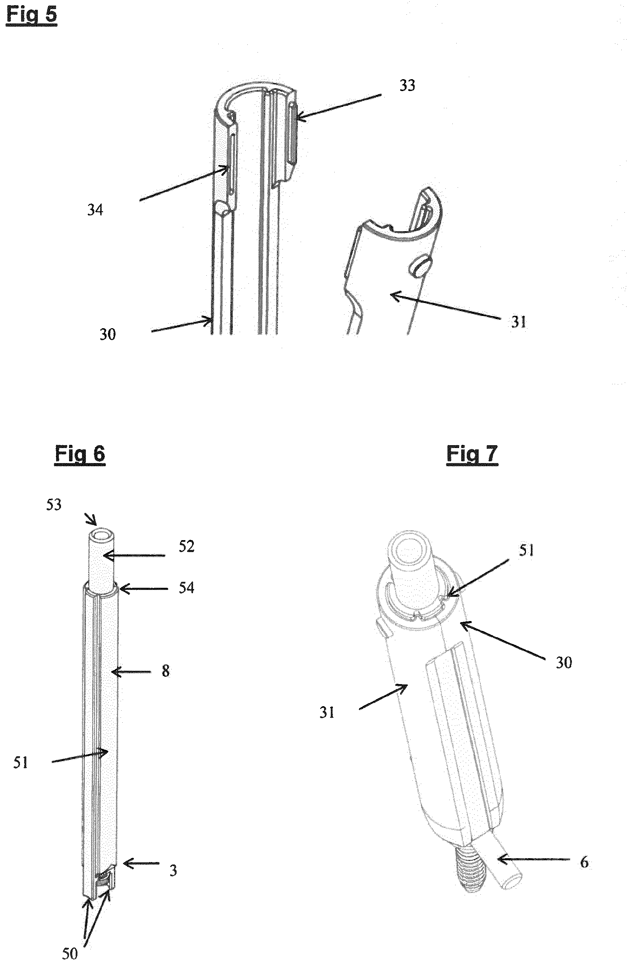

Avantageusement, le logement intérieur (21) est agencé pour permettre la mise en place de la demi-coquille par bascule/rotation de celle-ci sur la tête de vis (5) suivant la direction (A) avec comme point de rotation le logement intérieur (21). Cette forme d'empreinte male / femelle avec un logement intérieur (21) comporte l'avantage d'être indémontable quand les deux demi-coquilles sont jointives dans leurs parties distales (Figure 4a), et permet un démontage facile lorsque ces deux demi-coquilles (30 et 31) ne sont plus jointives (Figure 5).

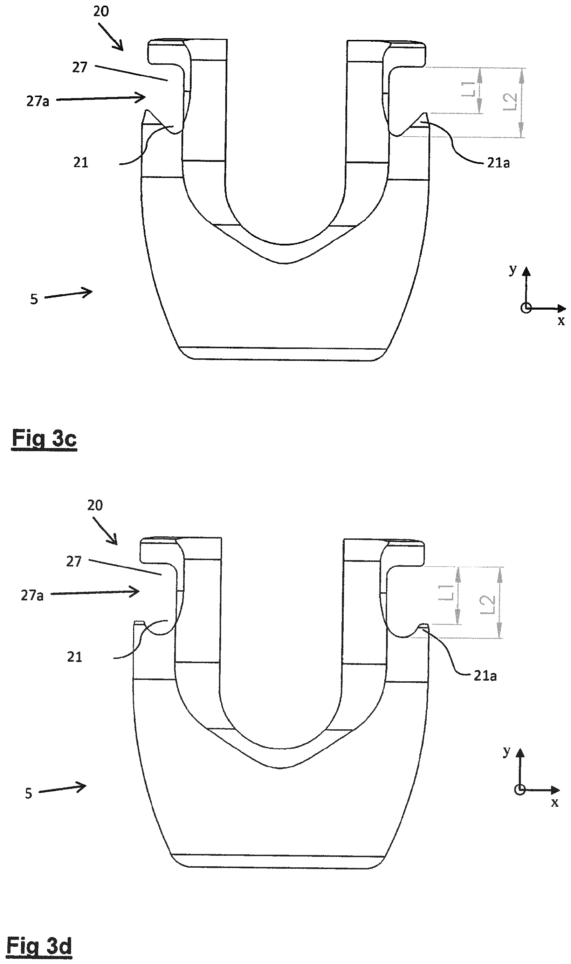

Les figures 3c et 3d illustrent respectivement deux exemples de forme d'empreintes. Dans les deux exemples illustrés, le canal (27) forme avec le logement (21) une cavité de réception présentant une hauteur interne L2 supérieure la hauteur L1 de l'ouverture d'entrée (27a). Cette différence dimensionnelle permet de bloquer l'extrémité proximale du tube de montage (7) sur la tête de vis (5). au moins en translation selon les axes (x, y).

Dans le mode de réalisation illustré, la différence dimensionnelle est portée sur le bas de l'empreinte, sous l'ouverture d'entrée (27a) afin de permettre un démontage des demi-coquilles (7) par basculement de ces dernières sur le rebord inférieur d'appui (21a) délimitant l'ouverture d'entrée (27a).

Dans une configuration particulière, le tube de montage (7) est surmoulé sur la vis.

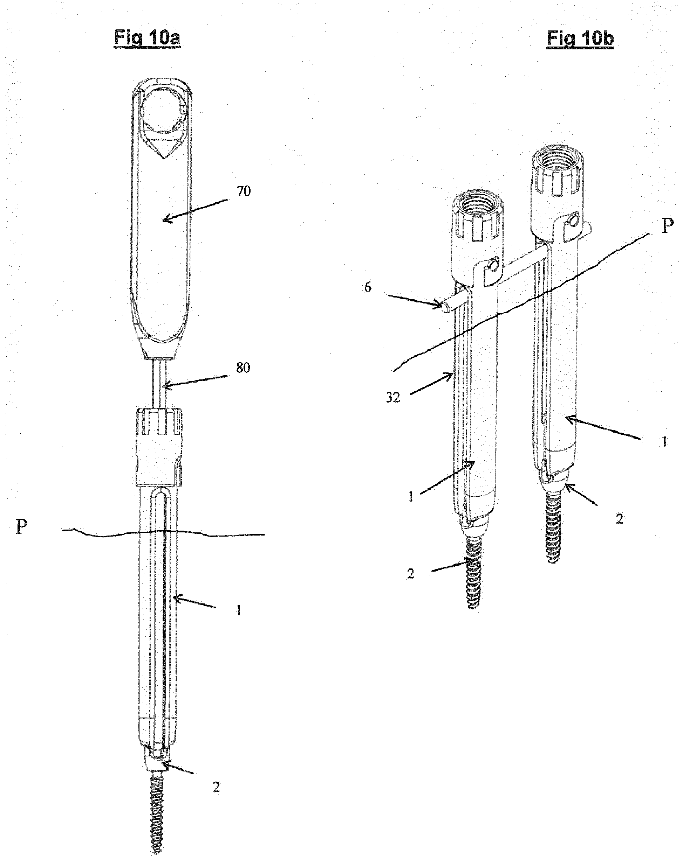

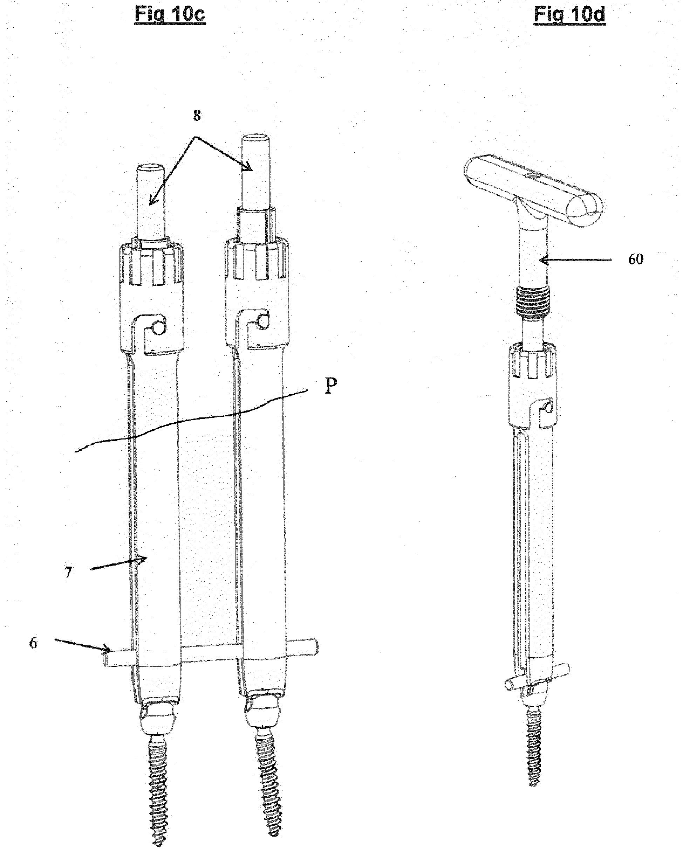

Avantageusement, le tube de montage (7) est pourvu d'une lumière (32) dans la continuité de la forme en U (25) de la tête de vis (5) afin de permettre le passage et l'introduction de la tige (6) (Figures 10b et 10c).

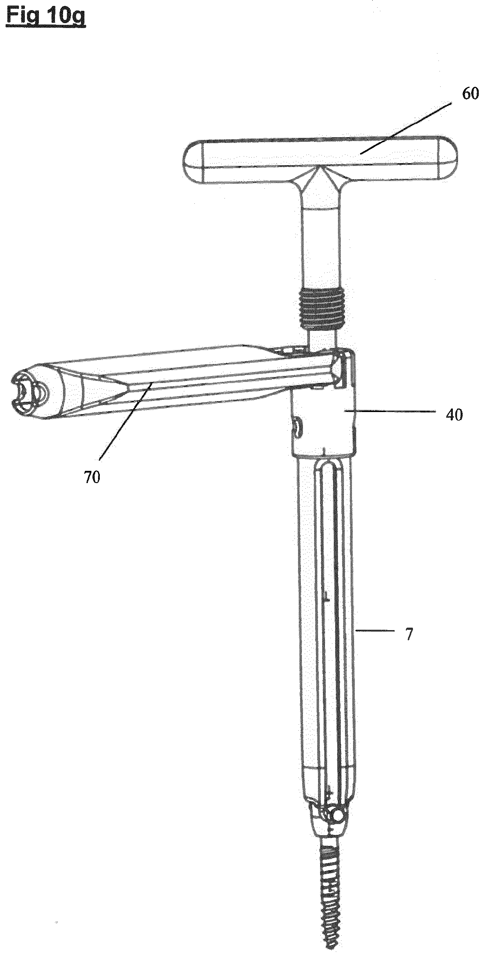

Avantageusement, la partie distale du tube (7) est pourvu d'un filetage (35), et d'une empreinte (36). Ce filetage (35) est prévu pour recevoir un élément de poussée (60) du tube porte élément de blocage (8), et l'empreinte (36) est prévue pour recevoir un élément perpendiculaire de maintien (70) (Figure 10g). Le filetage (35) est ménagé sur la surface opposée à la surface sur laquelle est ménagée l'empreinte. Dans le mode de réalisation décrit, le filetage est ménagé sur la surface interne de la partie distale du tube (7) et l'empreinte (36) sur la surface extérieure dudit tube.

Le tube de montage (7) n'est pas représenté dans sa version monobloc, mais uniquement dans les deux modes de réalisations préférés comme décrit ci-dessous. La forme extérieure du tube peut être de forme quelconque comme ovale ou encore hexagonale quelle qu'en soit la version.

La fixation du tube (7) sur l'implant d'ancrage osseux (2) peut être obtenue par différents designs non représentés. Le tube (7) peut être constitué de 2 bras formant pince, d'une fourche avec son système de fermeture de maintien à l'implant, ou encore de façon non limitation, d'un tube monobloc et de son moyen de fixation à l'implant, les moyens de connexion (21) de l'implant étant en adéquation avec le design du tube (7).

La figure 5 décrit dans un mode de réalisation préféré pour la jonction entre les deux demi-coquilles. Dans cette configuration les deux demi-coquilles (30 et 31) sont identiques, et s'interconnectent entre elles par un dispositif de tenon (33) / mortaise (34).

Dans une configuration particulière ces deux demi-coquilles peuvent être collées, soudées, clipsées, emboîtées, mais le mode de réalisation préféré consiste en une jonction par l'intermédiaire d'une bague (40) complémentaire (Figure 4a et 4b).

Dans ce cas, c'est la bague qui reçoit le filetage (35) et l'empreinte (36) prévus en l'extrémité distale du tube de montage (7). Avantageusement la forme intérieure de la bague correspond à la forme extérieure du tube de montage (7) ici composée des deux demi-coquilles.

Avantageusement dans le premier mode préféré de réalisation, la bague est pourvue d'au moins une baïonnette (41) dans l'épaisseur de la bague (40). Afin de solidariser la bague sur le tube de montage (7), celui-ci comporte au moins un pion (42) en vis-à-vis de la baïonnette(s). Afin de maintenir la bague (40) en place sur le tube de montage (7), un ressort de compression non représenté est intercalé entre les deux éléments. Le montage et le démontage de la bague et des deux demi-coquilles sont ainsi simplifiés.

Dans le second mode préféré de réalisation, la bague (40) est fixée par au moins un pion traversant (43). Lors des efforts de serrage du bouchon (3), ce pion est soumis aux efforts de cisaillement entre le couple nécessaire au serrage du bouchon (3) et le contre couple exercé par l'élément perpendiculaire de maintien (70) via l'empreinte (36). Avantageusement le pion traversant (43) est dimensionné pour rompre sous l'effort de cisaillement. Avantageusement le couple de rupture est fonction du couple optimal de serrage du bouchon (3). Avantageusement, la bague (40) est désolidarisée du tube (7) quand le serrage final de l'implant est effectué, et le démontage des deux demi-coquilles est ainsi simplifié.

Les figures 6 et 7 montrent le tube porte élément de blocage (8) pré-monté sur le bouchon (3), ainsi que son interconnexion dans le tube de montage (7) de la vis (2).

Le tube porte élément de blocage (8) est agencé pour maintenir de façon sécurisée le bouchon (3) dans sa partie proximale. L'ensemble tube porte élément de blocage (8) et le bouchon (3) constitue l'ensemble de blocage.

Avantageusement, le maintien du bouchon (3) est assuré par un taraudage agencé dans le tube porte élément de blocage (8).

Selon une variante particulière préférée, ce taraudage est légèrement différent du filetage du bouchon (3) afin d'assurer son maintien par coincement.

Selon une variante particulière, le maintien du bouchon (3) est assuré par un clipsage.

Avantageusement, l'extrémité distale de l'ensemble de blocage est pourvue de deux pattes (50) de part et d'autre du bouchon (3), d'une longueur au moins équivalente à la hauteur du bouchon par rapport à la face inférieure de celui-ci devant venir en contact de la tige (6). La largeur de ces pattes (50) ne peut être supérieure au diamètre de la tige (6). La fonction de ces deux pattes est de permettre l'appui sur la tige (6) sans contact du bouchon (3) ni sur la tête de la vis (5), ni sur la tige (6), jusqu'au contact de cette dite tige sur l'élément de solidarisation de la vis (5), soit le fond du canal en forme de U (25).

Selon une configuration particulière préférée, la forme extérieure du tube porte élément de blocage (8) est pourvue d'au moins une queue d'aronde (51) de forme complémentaire à la forme intérieure du tube de montage (7) de la vis (2). Avantageusement, le tube porte élément de blocage (8) est pourvu de deux queues d'aronde (51), une sur chaque demi-coquille.

Ces queues d'aronde (51) complémentaires entre les deux demi-coquilles (30 et 31) et le tube porte élément de blocage (8) renforcent mécaniquement l'ensemble et évitent ainsi tout risque d'écartement ou de désolidarisation sous contraintes élevées des deux demi-coquilles et donnent une résistance de l'ensemble supérieure en flexion et torsion.

Selon une configuration particulière préférée, la longueur totale du tube porte élément de blocage (8), est supérieure au tube de montage (7), ou bien aux deux demi-coquilles + bague. Il est ainsi possible de retirer le tube porte élément de blocage (8), une fois le bouchon (3) mis en place.

Selon cette configuration particulière, la partie distale (52) du tube porte élément de blocage (8) et la forme intérieure (62) de l'élément de poussée (60) sont compatible pour la mise en place de la tige (6) au fond du logement en forme de U (25), élément de solidarisation de la vis (2). Avantageusement cette forme (52) est cylindrique.

Avantageusement la poussée du tube (8) par l'élément de poussée (60) peut être réalisée en extrémité (53) de ce dit tube, ou bien sur l'épaulement (54) formé par l'aménagement de la forme (52).

Avantageusement, le diamètre intérieur du tube porte élément de blocage (8) est prévu pour le passage du tournevis.

Dans le mode de réalisation décrit, le tube porte élément de blocage (8) fait donc office également de pousse tige.

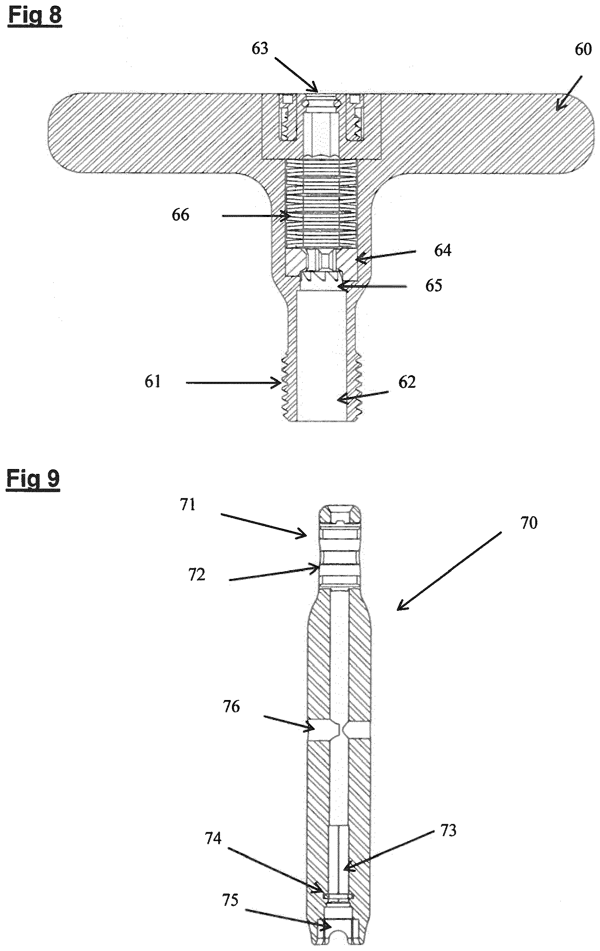

Avantageusement l'élément de poussée (60) du tube porte élément de blocage (8) / pousse tige est la poignée en T (60) qui est pourvue d'un filetage (61) correspondant à celui de l'extrémité distale du tube de montage (7) de la vis (2) comme montré dans les figures 8, 10 et 11.

Avantageusement cette poignée en T est canulée (63) sur toute sa longueur laissant passer au travers la barre de tournevis du bouchon (80').

Selon une configuration particulière, cette poignée (60) est dynamométrique réglée au couple de serrage optimum du bouchon (3) bloquant l'élément de liaison tige (6). Bien connu de l'homme du métier, le mécanisme dynamométrique est composé de deux bagues crantées (64) et (65), et d'une série de rondelles « Belleville » (66) ou ressort.

Selon cette configuration particulière, les parties proximales d'engrènement des différentes barres de tournevis (80 et 80') sont différentes afin de n'autoriser que la fonction dynamométrique sur la barre de tournevis destinée au serrage du bouchon.

Selon une autre configuration particulière non représentée, la barre tournevis (80') pour le serrage du bouchon (3) présente une amorce à la rupture de façon à rompre sous l'effort de serrage. Avantageusement le couple de rupture est fonction du couple optimal de serrage du bouchon (3). Dans cette configuration particulière, la barre de tournevis (80') peut être constituée en 2 parties interconnectées longitudinalement, un embout avec l'amorce à la rupture et la barre tournevis proprement dite. L'embout fait alors partie intégrante de l'ensemble de blocage (8 et 3).

La figure 9 montre l'élément perpendiculaire de maintien (70) du tube (7). Cet élément perpendiculaire de maintien (70) est pourvu sur l'une de ses extrémités d'une empreinte (71) complémentaire à celle de l'extrémité distale du tube de montage (7) de la vis (2). Dans la configuration préférée, l'empreinte (71) est formée par des encoches (72). Selon différentes configurations particulières, cette empreinte (71) peut prendre la forme d'un hexagone, d'un polygone, ou encore d'un hexalobe cités à titre d'exemple, le but recherché étant un maintien en rotation de l'élément perpendiculaire de maintien (70) sur le tube (7).

Avantageusement cet élément perpendiculaire de maintien (70) constitue également une poignée droite et permet de recevoir les différentes barres tournevis (80 et 80'). Une empreinte (73) est aménagée à ce titre.

Il peut être avantageux de prévoir également un moyen de maintien des barres tournevis (80 et 80') dans cette poignée (70). Dans la configuration préférée, ce maintien est assuré par un joint torique (74) de forme et taille compatibles aux diamètres des barres tournevis (80 et 80'). Ce maintien peut également être assuré par toute forme élastique, comme une languette par exemple (non représenté).

Avantageusement une empreinte complémentaire (76) est agencée sur le coté de la poignée , lorsqu'elle est considérée dans sa position droite (on parlera par la suite de poignée droite), afin que celle-ci puisse être utilisée comme une poignée en T compatible avec les barres tournevis (80 et 80').

Selon une configuration préférée, cette poignée droite (70) est pourvue dans une de ses extrémités d'une forme complémentaire (75) à la forme extérieure de la tête de la vis (5).

Cette poignée droite permet donc lors du serrage ou desserrage du bouchon d'effectuer un effet contre couple aussi bien quand le tube de montage (7) est en place que lors que celui-ci est démonté.

Avantageusement cette poignée droite est canulée sur toute sa longueur laissant passer au travers la barre tournevis bouchon (80').

Selon une configuration particulière, la poignée est agencée pour maintenir de façon sécurisée le bouchon de serrage (3) dans son extrémité proche de la forme complémentaire (75) à la tête de la vis (5). Cette possibilité permet de mettre en place le bouchon (3) dans la vis (2) si le tube de montage (7) n'est pas en place.

Avantageusement, ce maintien est assuré par un taraudage (76) de forme identique au bouchon (6), ou légèrement différent afin d'assurer le maintien du dit bouchon (6) par coincement.

Selon une autre variante particulière, le maintien du bouchon est assuré par un clipsage.

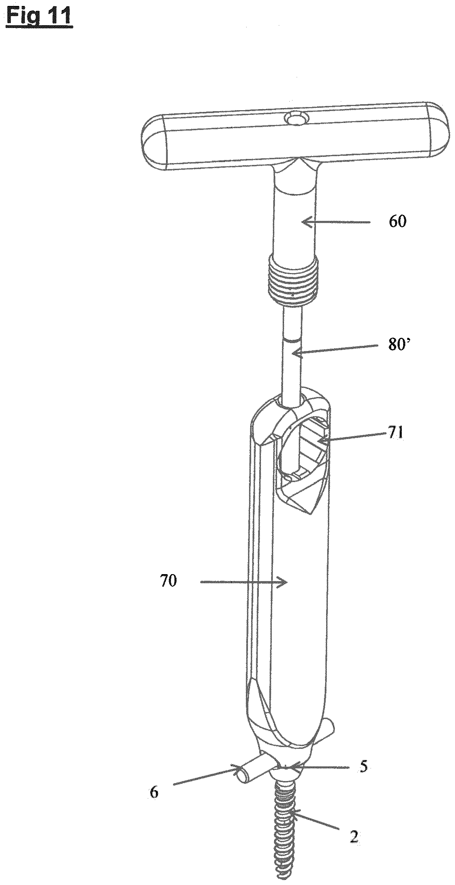

Les figures 10a à 10g et 11 décrivent les différentes étapes chirurgicales de l'invention (pour des raisons de simplicité, les vertèbres ne sont pas représentées):
- la figure 10a représente la mise place du dispositif vis (1) dans les vertèbres. La poignée (70) et la barre de tournevis (80) sont utilisées pour le vissage. Une bague complémentaire non représentée peut être utilisée pour la solidarisation de l'ensemble de ces éléments, par l'intermédiaire du filetage (35) du dispositif vis (1).
- la figure 10b représente deux dispositifs vis (1) avec la mise en place de la tige (6) en parties distales des tubes de montage (7) qui avantageusement se situent hors de la plaie du patient en delà de la peau ici représentée par la ligne (P).
- La figure 10c représente l'insertion de la tige (6) au travers des deux tubes de montage (7). Cette manœuvre de descente de la tige peut être effectuée soit à l'aide des tubes porte élément de blocage (8), soit par une pince porte tige de type ciseaux ou autre. Dans cette seconde configuration, la pince porte tige de type ciseaux non représentée, maintien la tige approximativement en son milieu de façon à passer entre les deux tubes de montage (7).
- La figure 10d représente la poussée de la tige (6) dans le canal en U de la vis (2) par l'intermédiaire de l'élément de poussée poignée (60). Le filet (61) situé dans la partie proximale de la poignée (60) permet d'exercer des efforts importants de poussée, tandis que les queues d'aronde situées dans les tubes (7) et (8) renforcent l'ensemble en torsion et flexion. Pour cette étape d'insertion de la tige (6), il est possible d'utiliser perpendiculairement l'élément de maintien (70) (non représenté) afin de contrecarrer le couple transmis pour la poussée, et ainsi avoir un bien meilleur contrôle du geste opératoire.

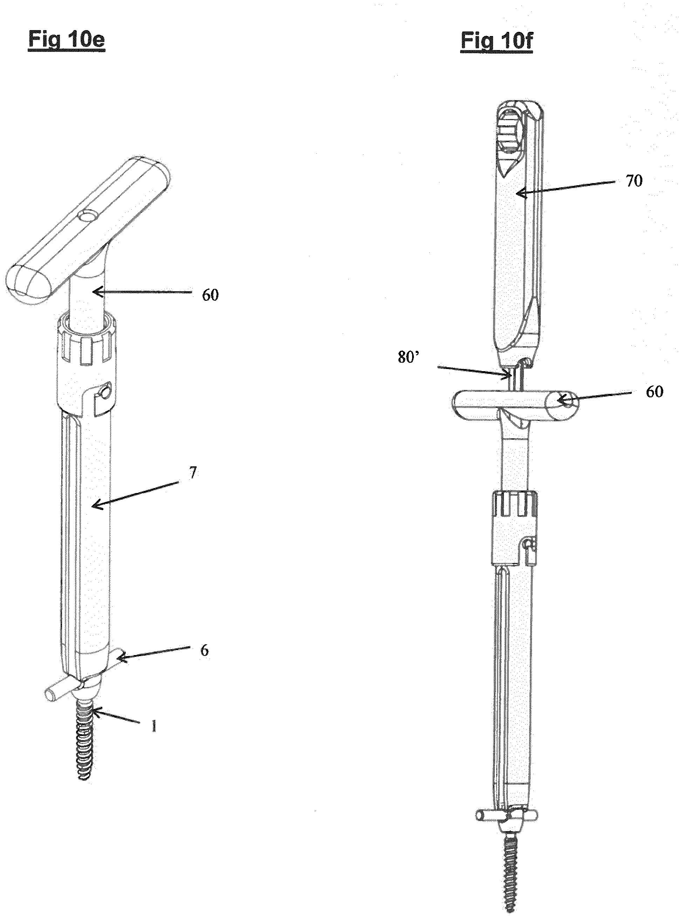

- La figure 10e représente la tige (6) en place en fond de la forme en U (25) de la tête de vis (5). Le bouchon (3) n'est pas encore engagé dans la tête de vis (5), et se trouve pré-monté sur le tube porte élément de blocage (8).
- La figure 10f représente la mise en place du bouchon (3) dans la tête de la vis (5) par l'intermédiaire de la poignée droite (70) et de la barre tournevis bouchon (80').
- La figure 10g représente l'étape de fixation finale de la tige (6) dans les vis (2). L'élément de maintien (70) assure perpendiculairement le contre couple et le maintien de l'ensemble, la poignée en T (60) et la barre tournevis bouchon (80') permettent d'exercer le couple de serrage du bouchon (3). Comme décrit précédemment la mesure du couple requis pour un serrage optimum peut être obtenue soit par rupture de la bague (40), soit par la poignée en T (60) de type dynamométrique, ou encore par une rupture de la barre tournevis (80').
- La figure 11 représente également l'étape finale de serrage si le tube de montage (7) n'est pas en place sur la vis (2). En effet pour des questions d'anatomie et d'encombrement, le chirurgien peut être amené à démonter le tube en cours de chirurgie. Cette éventualité est rare, mais parfois nécessaire pour la bonne conduite de la chirurgie. Dans cette configuration, le maintien contre couple de la tête de la vis (5) est assuré par la poignée (70) avantageusement pourvue d'une forme complémentaire à cette dite tête de vis (5) comme décrite précédemment. La poignée en T (60) de type dynamométrique et la barre tournevis bouchon (80') permettent d'exercer le couple de serrage du bouchon nécessaire pour la bonne tenue de l'implant. Dans cette configuration le tube de montage (7) peut être utilisé comme poignée perpendiculaire contre couple par l'intermédiaire de l'empreinte (71) (non représenté).
- Avantageusement cette configuration est utilisée pour le desserrage du bouchon, pour la dépose des implants, ou tout autre étape chirurgicale nécessitant la désolidarisation de la connexion vis (2) / tige (6).

Avantageusement, afin de réduire le nombre d'instruments nécessaires à la pose des implants la pince de compression et de distraction ne forme qu'une seule pince. Dans l'art de la technique, ces pinces sont composées de deux bras articulés, les parties proximales de ces pinces étant constituées de fourches venant chevaucher la tige (6), ces mêmes fourches venant exercer sur les implants, les efforts de compression / distraction voulus.

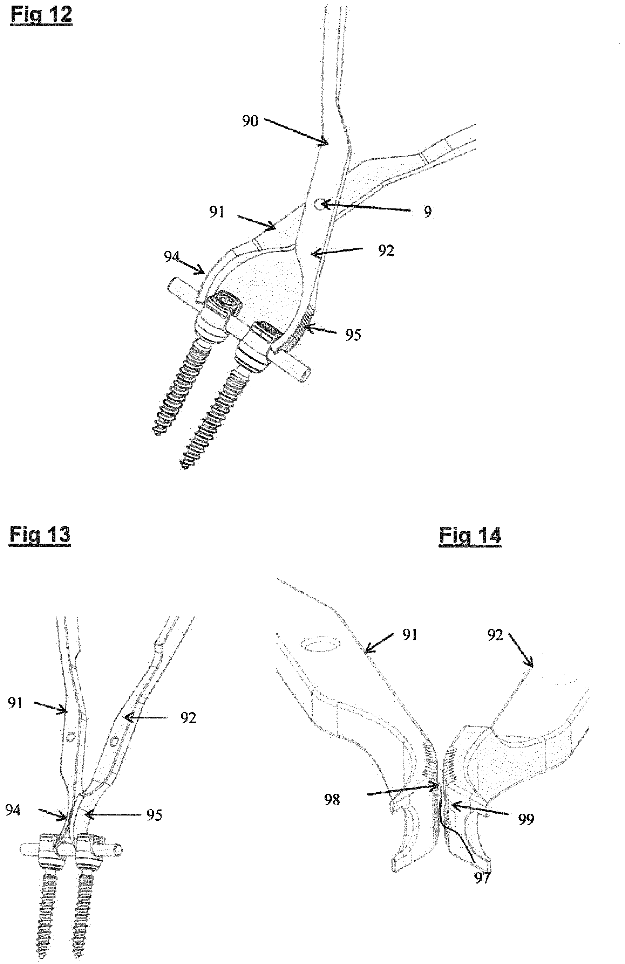

La figure 12 représente la pince compression (90). Cette pince est composée en principal de deux bras (91) et (92), et d'une articulation (93). Chaque bras (91) et (92) est pourvue d'une extrémité courbe (94) et (95) terminée par la dite forme de fourche.

Avantageusement, l'articulation (93) est facilement démontable.

La figure 13 représente ces mêmes bras (91) et (92) utilisés pour une manœuvre de distraction. Il est pris avantage de la forme courbe des deux extrémités proximales courbes (94) et (95), afin de celles-ci puissent « rouler » l'une sur l'autre, et ainsi exercer l'effort de distraction entre les vis (2) par compression de deux bras (91) et (92).

Dans la configuration préférée, deux extrémités proximales courbes (94) et (95) comportent un crantage dans leurs parties convexes afin de prévenir le glissement frontal des deux bras (91) et (92).

Avantageusement comme représentée dans la figure 14, les deux extrémités proximales courbes (94) et (95) comportent un moyen (97) d'interconnexion afin de prévenir le glissement latéral des deux bras (91) et (92).

Dans la configuration préférée, cette interconnexion est composée d'un pion (98) ménagé dans l'une des extrémités proximales courbes (94) ou (95), et d'une rainure (99) ménagée dans l'autre extrémité proximale courbes.

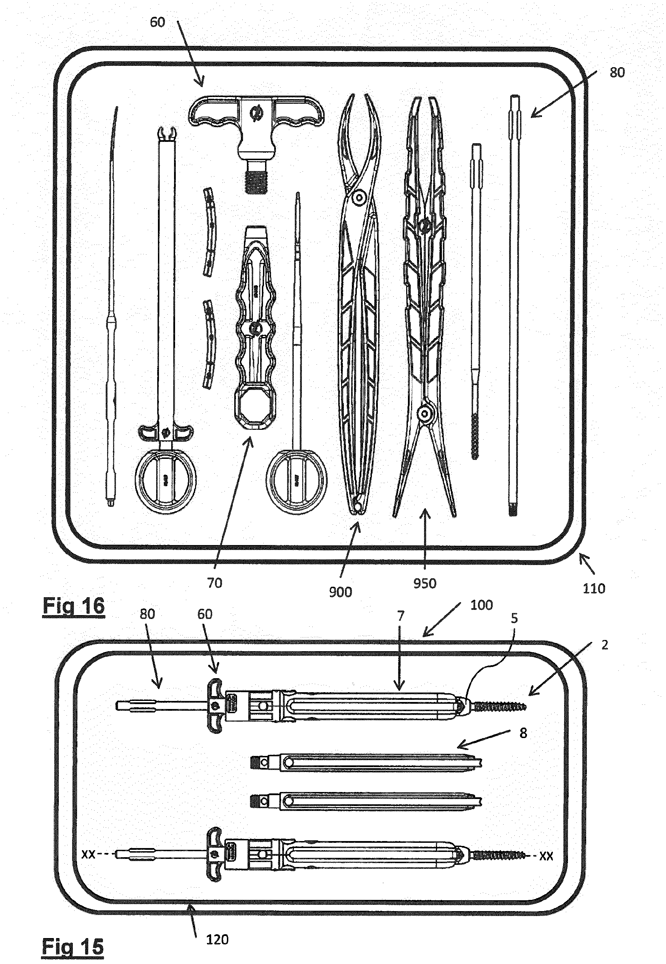

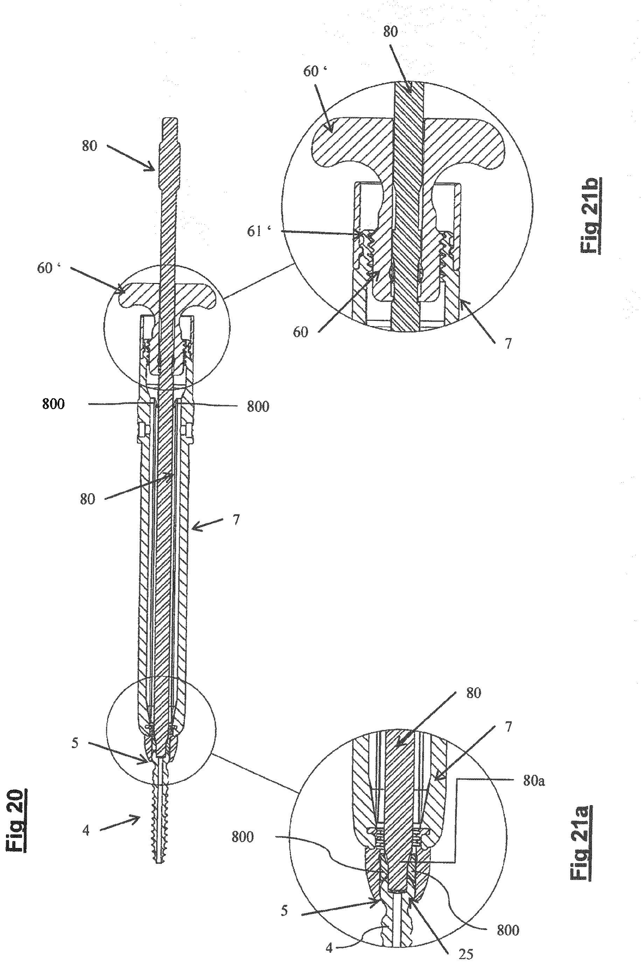

La figure 15 décrit un dispositif (100) pour la réalisation d'une stabilisation rachidienne selon un deuxième mode de réalisation. Dans ce mode de réalisation, le dispositif (100) comprend deux éléments d'ancrage osseux (2) pré-montés solidairement sur des tubes de montage (7) et deux éléments de blocage destinés à bloquer la tige de liaison (6) sur l'élément d'ancrage osseux, les éléments de blocage étant pré-montés sur des tubes de serrage (8). Avantageusement, les tubes (7) comportent une poignée (60) et un tournevis (80) pré-montés. L'agencement de ces éléments sera décrit plus loin, en relation avec les figures 20, 21a et 21b.

L'ensemble tubes/tournevis/poignées/vis et tubes/éléments de blocage sont disposés dans un emballage (120) scellé, de conditionnement stérile et à usage unique.

L'ensemble ainsi conditionné forme un kit d'ancrage osseux mettant à disposition, pour réaliser une stabilisation rachidienne à l'aide d'une tige de liaison, deux éléments d'ancrage osseux et deux éléments de blocage.

Il est bien entendu évident qu'il peut être prévu des kits d'ancrage osseux comprenant plus de deux éléments d'ancrage osseux et deux éléments de blocage pré-montés sur des tubes comme décrit ci-dessus sans pour autant sortir du champ de l'invention.

La figure 16 décrit un kit d'instrument (110) pour la pose ou la dépose d'implants selon un deuxième exemple de réalisation de l'invention. Comme dans l'exemple de réalisation précédemment décrit (figure 2), le kit d'instruments (110) est à usage unique conditionné stérile.

Dans le mode de réalisation illustré, le kit d'instrument (110) comporte des instruments de préparation des trous pédiculaires (spatule, palpeur taraud), ainsi que des instruments nécessaires à l'implantation des vis pédiculaires et tiges de liaison (barre de tournevis 80, pinces 900, 950 et poignées 60, 70). Il a noté que les pinces (900, 950) présentes dans le kit d'instruments (110) présentent une double fonctionnalité qui sera décrite plus loin.

Le kit d'instruments (110) reprend l'ensemble des caractéristiques pour le kit d'instruments (10) précédemment décrit.

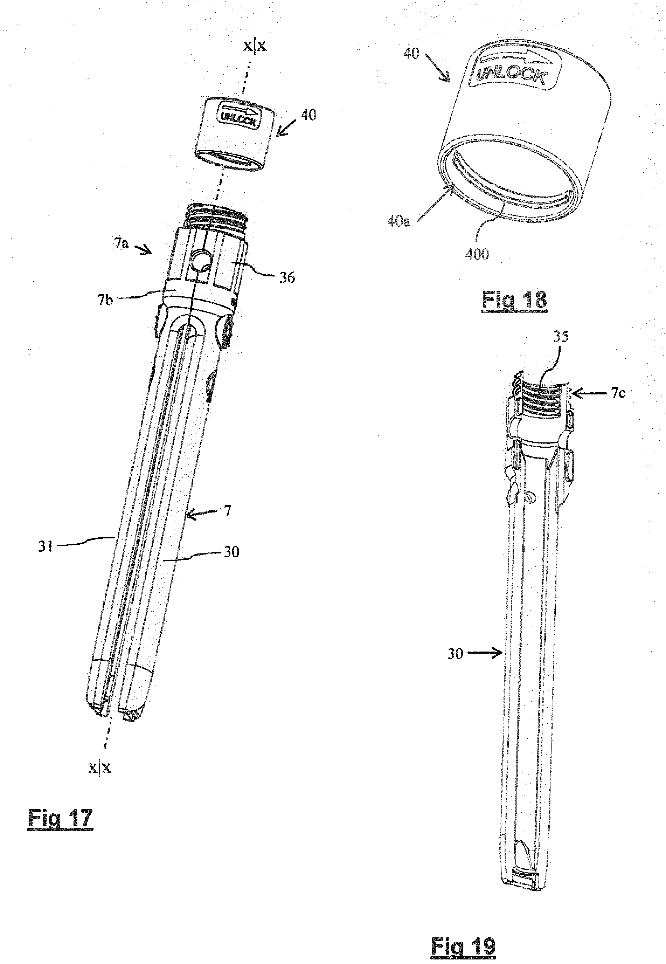

Les figures 17 à 19 représentent des vues d'un tube (7) et d'une bague de maintien (40) formant de dispositif de la figure 15. Le tube (7) reprend l'ensemble des caractéristiques du tube décrit précédemment. Cependant, dans l'exemple illustré sur la figure 15, les moyens mis en œuvre pour recevoir un élément de poussée (ou poignée en T) (60) ainsi qu'un élément perpendiculaire de maintien (70) portés, dans l'exemple précédemment décrit, par la bague 40, sont à présent portés directement par le tube (7).

Ainsi, dans le mode de réalisation décrit, la partie distale (7a) du tube (7) comporte une face extérieure (7b) pourvue de l'empreinte (36) destinée à recevoir l'élément perpendiculaire de maintien (70) et une face intérieure (7c) pourvue du filetage (35) pour recevoir l'élément de poussée (60). Dans le mode de réalisation décrit, et comme dans l'exemple précédent, l'empreinte a une forme hexagonale.

Avantageusement, la partie distale (7a) du tube (7) comporte, dans le prolongement de l'empreinte (36), un filetage externe (7d) apte à coopérer avec au moins une languette (400) de forme complémentaire ménagée sur la face interne (40a) de la bague (40), permettant ainsi la fixation de la bague en extrémité distale du tube (7). Dans cet exemple de réalisation, la bague (40) a pour seule fonction le maintien des deux demi-coquilles (30, 31) l'une par rapport à l'autre.

Avantageusement, le filetage externe ménagé sur chaque demi-coquille (30, 31) est constitué d'un double filet de vissage afin de s'assurer que le filetage soit identique sur chaque demi-coquille de manière à ce que le filetage résultant de la jonction des filetages des deux demi-coquilles soit continu.

Avantageusement, la bague (40) présente un diamètre extérieur inférieur au diamètre nominal de l'empreinte (36) lorsque les deux demi-coquilles (30, 31) sont en position pour former le tube (7). Ce dimensionnement spécifique de la bague (40) a pour but de permettre le passage de l'élément perpendiculaire de maintien (70) pour son position au niveau de l'empreinte (36).

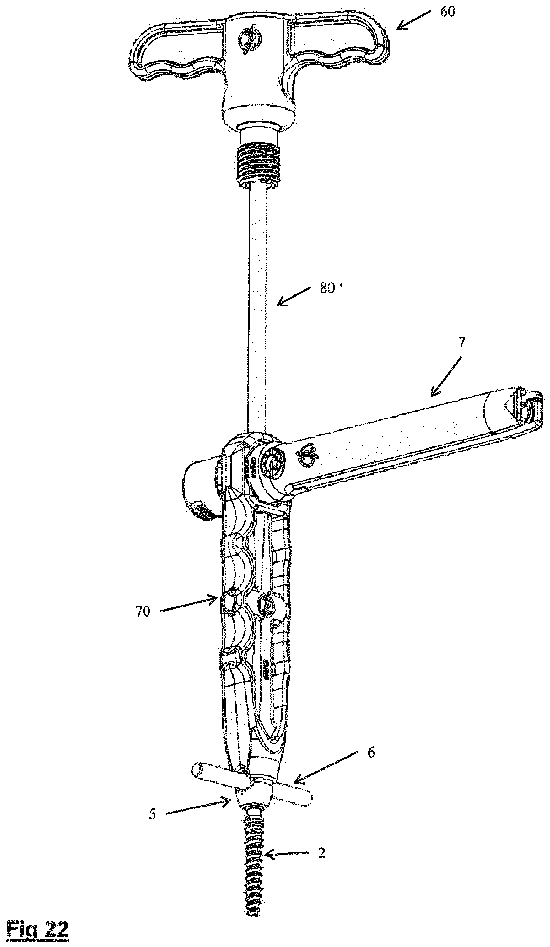

Avantageusement, la partie distale (7a) comporte un trou (700) ménagé au niveau de l'empreinte (36) pour permettre le passage d'une barre de tournevis (80, 80') lorsque l'élément de maintien (70) est utilisé en substitution du tube (7), comme illustré sur la figure 22. Dans ce mode de réalisation illustré, le tube (7) est utilisé en remplacement de l'élément de maintien (70), le tube ayant ainsi une fonction de poignée, et l'élément de maintien (70) en remplacement du tube (7).

La figure 20 illustre une vue en coupe selon l'axe XX-XX de l'ensemble tube de montage/ tournevis/ élément d'ancrage illustré sur la figure 15.

Comme illustré sur les figures 20 et 21a, la barre de tournevis (80) est pré-montée sur l'élément d'ancrage (2). Plus particulièrement, l'extrémité proximale (80a) de la barre de tournevis (80) est agencée pour coopérer avec la vis (2). Avantageusement, l'ensemble comporte des moyens permettant de maintenir la barre de tournevis (80) dans l'axe de la partie filetée (4) de l'élément d'ancrage (2). A titre d'exemple non limitatif, les moyens de maintien de la barre de tournevis (80) dans l'axe de l'élément d'ancrage osseux comprennent des ergots (800) ménagés sur la face interne du tube (7).

Comme illustré sur les figures 20 et 21b, l'ensemble comporte avantageusement une ailette de maintien (60') de la barre de tournevis 80 en position donnée par rapport au tube (7) et à la vis (2).

L'ailette de maintien (60'), en forme de T, est pré-montée sur l'extrémité distale du tube (7). Elle comporte, dans sa partie inférieure, un filetage (61') correspondant au taraudage (35) de la partie distale du tube (7).

Avantageusement, l'ailette de maintien (60') comporte, sur toute sa longueur, un canal traversé par la barre de tournevis (80).

L'ailette de maintien (60') est agencée pour assurer une retenue axiale de la barre de tournevis (80) dans le tube (7) tout en autorisant un mouvement de rotation de la barre à l'intérieur du tube (7) et ce afin de permettre l'entrainement en rotation de la partie filetée (4) de la vis (2), la tête (5) de la vis restant fixe par rapport à la barre.

Selon un mode de réalisation avantageux, elle est agencée pour conserver l'alignement de la barre de tournevis (80) dans l'alignement de l'élément d'ancrage osseux (2).

Du fait d'un pré-montage en usine de la barre de tournevis (80) sur la vis également pré-montée dans un tube (7), une tolérance réduite est autorisée, et de fait il est assuré un meilleur maintien de la vis suivant des efforts de flexion.

Un tel pré-montage a également pour avantage de permettre d'une part un gain de temps chirurgical, l'opération de mise en place de la barre de tournevis sur l'élément d'ancrage osseux étant supprimée et d'autre part une limitation du risque opératoire, la durée d'anesthésie étant réduite.

Un tel pré-montage a également pour avantage d'éviter tout problème d'appairage barre de tournevis/vis.

De même que dans le mode de réalisation illustré sur la figure 11, le tube (7) peut être utilisé comme poignée perpendiculaire et la poignée perpendiculaire (70) comme tube de guidage d'une barre de tournevis bouchon (80') (figure 22).

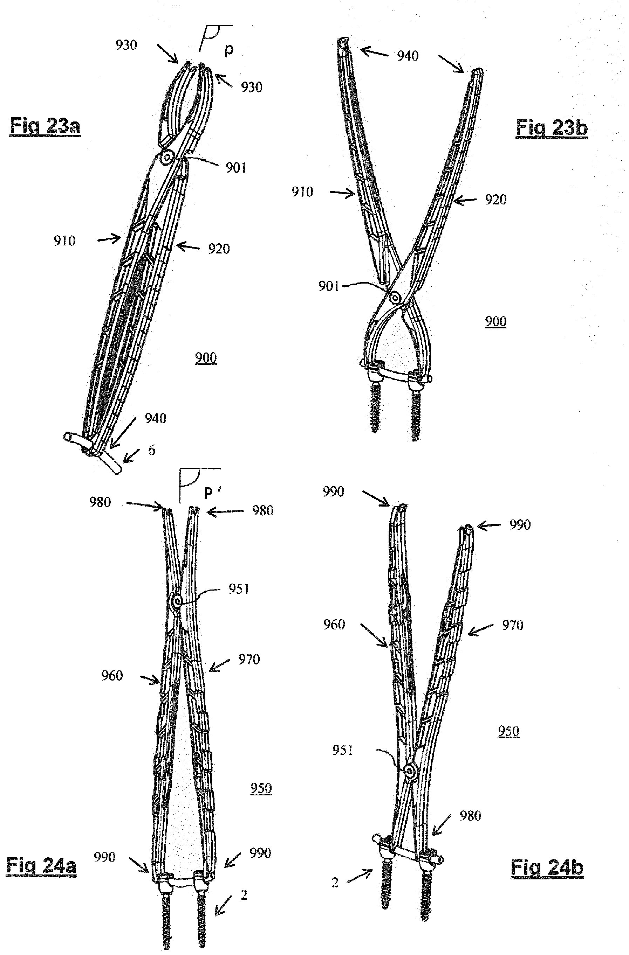

Les figures 23a et 23b représentent une pince de compression (900) contenue dans le kit d'instruments (110). La pince de compression (900), laquelle présente un plan de symétrie P, comporte deux bras (910), (920) montés articulés l'un par rapport à l'autre. Dans le mode de réalisation décrit, les bras sont disposés croisés l'un par rapport à l'autre.

Comme pour la pince de compression précédemment décrite (figure 12), chaque bras (910, 920) présente une extrémité courbe (930) en forme de fourche, les extrémités (930) en forme de fourche de chaque bras étant disposées en vis-à-vis l'une de l'autre par rapport au plan de symétrie de sorte à pourvoir venir chevaucher la tige (6). Les extrémités des bras ainsi agencées forment des extrémités de la pince dites extrémités de compression (930).

Avantageusement, les extrémités opposées (940) aux extrémités de compression (930) sont agencées pour permettre la préhension de la tige (6) par rapprochement des bras (910, 920). On parlera par la suite d'extrémités de préhension (940) de la pince (900). Afin de faciliter la préhension, les extrémités de préhension (940) de chaque bras (910, 920) comportent deux doigts délimitant un espace de réception de la tige (6). Ainsi, lorsque les bras (910, 920) sont en position rapprochée, la tige (6) est maintenue enserrée entre les doigts des extrémités de préhension (940) de chacun des bras.

Ainsi configurée, la pince de compression (900) permet au moyen de ses extrémités de compression (930) d'exercer un effort de compression sur les éléments d'ancrage osseux, comme illustré sur la figure 23b, et au moyen de ses extrémités de préhension (940), opposées aux extrémités de compression, de tenir la tige, comme illustrée sur la figure 23a. Cette double fonctionnalité permet de réduire le nombre d'instruments nécessaire à la pose de l'implant et donc de réduire d'autant le nombre d'instruments à manipuler.

Afin de réduire l'effort nécessaire pour exercer une compression des implants, l'articulation (901) est prévue pour que la distance entre les extrémités de compression (930) et l'articulation (901) soit inférieure à la distance entre les extrémités de préhension (940) et l'articulation (901), la distance étant considérée selon l'axe longitudinal de la pince.

Les figures 24a et 24b représentent une pince de distraction (950) contenue dans le kit d'instruments (100).

Dans ce mode de réalisation, la pince de distraction (950), laquelle présente un plan de symétrie P', comporte deux bras (960), (970) joints l'un à l'autre par une articulation (951).

Chaque extrémité (980) de chaque bras (960, 970), configurée en forme de fourche, est agencée pour pourvoir venir chevaucher une tige (6) comme illustré sur les figures 24a et 24b. Ainsi, suivant leur positionnement sur la tige (6), selon qu'elles soient placées entre les deux éléments d'ancrage (2) (figure 24b) ou bien de part et d'autre des deux éléments d'ancrage (2) (figure 24a), les extrémités pourront exercer des efforts de distraction/compression souhaités sur la tige (6). On parlera alors d'extrémités de distraction (980) ou d'extrémités de compression (990).

Cette double fonctionnalité permet de réduire le nombre d'instruments nécessaire à la pose de l'implant et donc de réduire d'autant le nombre d'instrument à manipuler.

Afin d'autoriser des compressions de grande distance, l'articulation (951) est prévue pour que la distance entre les extrémités de distraction (980) et l'articulation (951) soit inférieure à la distance entre les extrémités de compression (990) et l'articulation (951), la distance étant considérée selon l'axe longitudinal de la pince.

Ainsi configurée, la pince de distraction (950) permet, au moyen de ses extrémités, d'exercer un effort de distraction sur les implants, comme illustré sur la figure 24b, et au moyen des extrémités opposées, d'exercer un effort de compression de grande amplitude, comme illustrée sur la figure 24a.

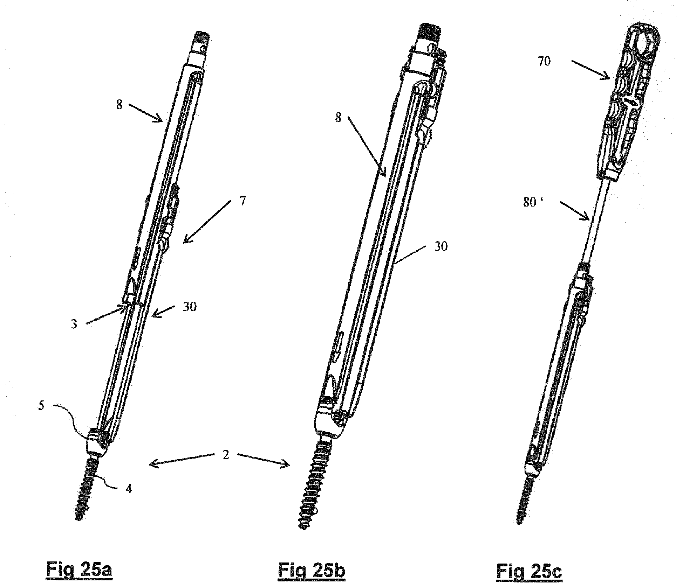

Les figures 25a à 25c illustrent les étapes de montage d'un bouchon (3) sur la tête (5) de vis (2) alors que le tube de montage (7) a été démonté de la vis (2).

Ainsi, et comme illustré sur la figure 25a, le tube (8) portant le bouchon (3) est mis en place sur la tête (5) de vis par glissement du tube (8) le long d'une des demi-coquilles (30, 31) montée sur la tête (5) de vis. La demi-coquille (30) permet ainsi de guider le bouchon (3) vers la tête de vis.

Une fois le tube (8) entièrement logé dans la demi-coquille (30) (figure 25b), la barre de tournevis bouchon (80') est insérée dans le conduit du tube de blocage (8) pour procéder au serrage du bouchon (3) sur la tige (6) (non représentée sur les figures mais présente) et sur la tête (5) de vis (figure 25c).

Le mode de réalisation illustré sur les figures 25a à 25c est avantageux en ce sens qu'une seule demi-coquille du tube (7) est nécessaire pour permettre le guidage et la mise en place du bouchon (3) sur la tête (5).

## Revendications

1. Dispositif (1, 100) rachidien pour la fixation des vertèbres par voie postérieure ou postéro-latérale chirurgicale minimalement invasive, le dispositif (1) comprenant au moins :
- un élément d'ancrage osseux (2) de type vis pédiculaire ou vertébrale, comportant une partie proximale (5) pourvue d'une interface pour solidariser un élément de liaison (6) de type tige ou plaque et d'une partie distale filetée (4),
**caractérisé en ce qu'**il comprend
- un tube de montage (7) pré-monté solidairement sur l'élément d'ancrage osseux (2), le tube de montage (7), composé de deux demi-coquilles (30, 31) présentant chacune une extrémité proximale en prise avec l'élément d'ancrage osseux (2) et étant maintenues solidairement l'une à l'autre au niveau de leur extrémité distale et jointives dans leurs parties distales, étant démontable de l'élément d'ancrage osseux (2), et
- une barre de tournevis (80) traversant le tube de montage (7) et présentant une extrémité pré-montée sur l'élément d'ancrage osseux (2), et
- un emballage scellé de conditionnement stérile (1) de l'élément d'ancrage osseux (2) et du tube de montage (7) et de la barre de tournevis pré-montés.

2. Dispositif (1, 100) rachidien pour la fixation des vertèbres selon la revendication 1, **caractérisé en ce que** le tube de montage (7) comporte une ouverture longitudinale (32) débouchant en partie proximale (5) de l'élément d'ancrage osseux (2) pour le passage de l'élément de liaison (6).

3. Dispositif (1, 100) rachidien pour la fixation des vertèbres selon la revendication 1 ou la revendication 2, **caractérisé en ce que** le dispositif rachidien comporte en outre un élément de blocage (3), le diamètre intérieur du tube de montage (7) permettant le passage de l'élément de blocage (3) de l'élément de liaison sur l'élément d'ancrage osseux.

4. Dispositif (1, 100) rachidien pour la fixation des vertèbres selon l'une quelconque des revendications 1 à 3, **caractérisé en ce que** le tube de montage (7) est pourvu de moyens permettant de maintenir l'ancillaire dans l'alignement de l'élément d'ancrage osseux (2).

5. Dispositif (1, 100) rachidien pour la fixation des vertèbres selon la revendication 4, **caractérisé en ce que** les moyens de maintien comprennent des ergots ménagés sur la face interne du tube de montage (7).

6. Dispositif (1, 100) rachidien pour la fixation des vertèbres selon la revendication 4 ou la revendication 5, **caractérisé en ce que** les moyens de maintien comprennent une ailette de maintien (60') montée sur l'extrémité du tube de montage (7) opposée à l'extrémité portant l'élément d'ancrage osseux (2).

7. Dispositif (1, 100) rachidien pour la fixation des vertèbres selon la revendication 6, **caractérisé en ce que** l'ailette de maintien (60') est agencée avec le tube de montage (7) pour permettre un mouvement de rotation de l'ancillaire à l'intérieur du tube de montage (7).

8. Dispositif (1, 100) rachidien pour la fixation des vertèbres selon l'une quelconque des revendications précédentes, **caractérisé en ce que** le tube de montage (7) est réalisé en matière composite, polymère, alliage ferreux ou non ferreux ou encore une combinaison de ses différentes matières.

9. Dispositif (1, 100) rachidien pour la fixation des vertèbres selon l'une quelconque des revendications précédentes, **caractérisé en ce que** le tube de montage (7) est surmoulé sur l'élément d'ancrage osseux.

10. Dispositif (1, 100) rachidien pour la fixation des vertèbres selon l'une quelconque des revendications précédentes, **caractérisé en ce que** le tube de montage (7) comporte en extrémité distale une empreinte (36) pour la mise en place d'un élément de maintien (70) perpendiculaire de type poignée.

11. Dispositif (1, 100) rachidien pour la fixation des vertèbres selon l'une quelconque des revendications précédentes, **caractérisé en ce que** les deux demi-coquilles sont maintenues l'une à l'autre au niveau de leur extrémité distale par l'intermédiaire d'une bague (40).

12. Dispositif (1, 100) rachidien pour la fixation des vertèbres selon la revendication 11, **caractérisé en ce que** le tube de montage (7) et la bague (40) sont agencés pour former un ensemble rigide.

13. Dispositif (1, 100) rachidien pour la fixation des vertèbres selon l'une quelconque des revendications 1 à 12, **caractérisé en ce que** les deux demi-coquilles (30, 31) sont agencées pour former, lorsqu'elles sont maintenues solidairement l'une à l'autre, un tube de guidage.

14. Dispositif (1, 100) rachidien pour la fixation des vertèbres selon la revendication 13, **caractérisé en ce que** le tube de guidage formé par les deux demi-coquilles présente un diamètre intérieur permettant le passage d'un élément de blocage (3) de l'élément de liaison sur l'élément d'ancrage osseux (2).

15. Dispositif (1, 100) rachidien pour la fixation des vertèbres selon la revendication 13 ou la revendication 14, **caractérisé en ce que** le tube de guidage formé par les deux demi-coquilles présente un diamètre intérieur permettant le passage d'ancillaires assurant la mise en place de l'élément de blocage.

16. Dispositif (1, 100) rachidien pour la fixation des vertèbres selon l'une quelconque des revendications 3 à 15, **caractérisé en ce que** l'élément de blocage (3) est pré-monté solidairement avec un tube porte élément de blocage (8) pour former un ensemble de blocage, l'élément de blocage (3) étant démontable du tube porte élément de blocage (8).

17. Dispositif (1, 100) rachidien pour la fixation des vertèbres selon la revendication 16, **caractérisé en ce que** l'ensemble de blocage est conditionné stérile.

18. Dispositif (1, 100) rachidien pour la fixation des vertèbres selon la revendication 16 ou la revendication 17, **caractérisé en ce que** l'ensemble de blocage est conditionné stérile dans l'emballage scellé de conditionnement (1).

19. Dispositif (1, 100) rachidien pour la fixation des vertèbres selon l'une quelconque des revendications 16 à 18, **caractérisé en ce que** le tube porte élément de blocage (8) présente des dimensions permettant son insertion dans le tube de montage (7).

20. Dispositif (1, 100) rachidien pour la fixation des vertèbres selon la revendication 19, **caractérisé en ce que** le tube de montage (7) présente une paroi intérieure tubulaire pourvue d'au moins un relief d'indexation, le tube porte élément de blocage présentant un relief d'indexation complémentaire de forme complémentaire au relief d'indexation du tube de montage (7).

21. Dispositif (1, 100) rachidien pour la fixation des vertèbres selon l'une quelconque des revendications 16 à 20, **caractérisé en ce que** le tube porte élément de blocage (8) présente une partie proximale pourvue d'un relief d'appui, situé en regard de l'interface de solidarisation de l'élément d'ancrage osseux (2) lorsque le tube porte élément de blocage (8) est inséré dans le tube de montage (7), pour appuyer un élément de liaison (6) sur l'interface de solidarisation de l'élément d'ancrage osseux (2).

22. Dispositif (1, 100) rachidien pour la fixation des vertèbres selon l'une quelconque des revendications 16 à 21, **caractérisé en ce qu'**il comporte en outre un élément de poussée (60) coopérant avec le tube porte élément de blocage (8) pour insérer le tube porte élément de blocage (8) dans le tube de montage (7).

23. Dispositif (1, 100) rachidien pour la fixation des vertèbres selon l'une quelconque des revendications 16 à 22, **caractérisé en ce que** le tube de montage (7), lorsque le tube porte élément de blocage (8) est positionné en son sein, forme un tube de guidage.

24. Dispositif (1, 100) rachidien pour la fixation des vertèbres selon l'une quelconque des revendications 16 à 23, **caractérisé en ce que** le tube de montage (7) et le tube porte élément de blocage (8), lorsqu'il est positionné en son sein, forment un ensemble rigide.

25. Dispositif (1, 100) rachidien pour la fixation des vertèbres selon l'une quelconque des revendications 16 à 24, **caractérisé en ce que** le tube porte élément de blocage (8) présente une forme intérieure permettant le passage d'un tournevis.

26. Dispositif (1, 100) rachidien pour la fixation des vertèbres selon l'une quelconque des revendications 16 à 25, **caractérisé en ce que** le tube porte élément de blocage (8) est réalisé en matière composite, polymère, alliage ferreux ou non ferreux ou encore une combinaison de ses différentes matières.

27. Dispositif (1, 100) rachidien pour la fixation des vertèbres selon l'une quelconque des revendications précédentes, **caractérisé en ce que** toutes les pièces du dispositif sont à usage unique.

28. Dispositif (1, 100) rachidien pour la fixation des vertèbres selon l'une quelconque des revendications précédentes, **caractérisé en ce que** toutes les pièces du dispositif sont conditionnés de façon stérile dans un ou plusieurs emballages scellés (1, 10).

## Patentansprüche

1. Vorrichtung (1, 100) zur Befestigung von Wirbeln auf dem minimal-invasive chirurgische posterioren oder posterior-lateralen Weg, wobei die Vorrichtung (1) mindestens folgendes umfasst:
- ein Knochenverankerungselement (2), wie zum Beispiel eine Pedikelschraube oder Wirbelschraube, welches einen proximalen Teil (5) aufweist, der mit einer Grenzfläche versehen ist, um ein Verbindungselement (6), wie zum Beispiel eine Stange oder ein Plättchen, mit einem distalen Teil mit Gewinde (4) zu verbinden,
**dadurch gekennzeichnet, dass** sie Folgendes umfasst:
- ein fest auf dem Knochenverankerungselement (2) vormontiertes Montagerohr (7), wobei sich das Montagerohr (7), bestehend aus zwei Halbschalen (30, 31), jeweils ein proximales Ende aufweisen, dass so angeordnet ist, dass es in das Knochenverankerungselement (2) eingreift und an ihrem distalen Ende fest aneinander liegen und die an ihren distalen Teilen aneinander liegen, vom Knochenverankerungselement (2) abmontieren lässt, und
- ein Schraubendreherstab (80) umfasst, welches durch das Montagerohr (7) führt und auf dem Knochenverankerungselement (2) ein vormontiertes Endstück aufweist
- eine versiegelte Verpackung zum sterilen Verschluss (1) des Knochenverankerungselements (2) und des Montagerohrs (7) und des Schraubendreherstab (80) vormontierten.

2. Vorrichtung (1, 100) zur Befestigung von Wirbeln nach Anspruch 1, **dadurch gekennzeichnet, dass** das Montagerohr (7) eine längliche Öffnung (32) aufweist, die zum proximalen Teil (5) des Knochenverankerungselements (2) führt, um das Verbindungselement (6) hindurchführen zu können.

3. Vorrichtung (1, 100) zur Befestigung von Wirbeln nach Anspruch 1 oder Anspruch 2, **dadurch gekennzeichnet, dass** die Vorrichtung des Weiteren ein blockierendes Element (3) aufweist, wobei der Innendurchmesser des Montagerohrs (7) die Durchführung des blockierenden Elements (3) des Verbindungselements bis auf das Knochenverankerungselement ermöglicht.

4. Vorrichtung (1, 100) zur Befestigung von Wirbeln nach einem der Ansprüche 1 bis 3, **dadurch gekennzeichnet, dass** das Montageohr (7) mit Mitteln versehen ist, die das Hilfsmittel in der Ausrichtung des Knochenverankerungselements (2) halten.

5. Vorrichtung (1, 100) zur Befestigung von Wirbeln nach Anspruch 4, **dadurch gekennzeichnet, dass** die Haltemittel an der Innenseite des Montagerohrs (7) Stifte aufweisen.

6. Vorrichtung (1, 100) zur Befestigung von Wirbeln nach Anspruch 4 oder 5, **dadurch gekennzeichnet, dass** die Haltemittel einen Halteflügel (60') umfassen, der auf dem Endstück des Montagerohrs (7) gegenüber dem Endstück mit dem Knochenverankerungselement (2) montiert ist.

7. Vorrichtung (1, 100) zur Befestigung von Wirbeln nach Anspruch 6, **dadurch gekennzeichnet, dass** der Halteflügel (60') in Bezug auf das Montagerohr (7) so angeordnet ist, dass eine Drehbewegung des Hilfsmittels im Innern des Montagerohrs (7) möglich ist.

8. Vorrichtung (1, 100) zur Befestigung von Wirbeln nach einem der vorhergehenden Ansprüche, **dadurch gekennzeichnet, dass** das Montagerohr (7) aus Verbundmaterial, Polymer, Eisen- oder Nichteisenlegierung oder einer Kombination dieser Materialien hergestellt ist.

9. Vorrichtung (1, 100) zur Befestigung von Wirbeln nach einem der vorhergehenden Ansprüche, **dadurch gekennzeichnet, dass** das Montagerohr (7) auf das Knochenverankerungselement aufgeformt ist.

10. Vorrichtung (1, 100) zur Befestigung von Wirbeln nach einem der vorhergehenden Ansprüche, **dadurch gekennzeichnet, dass** das Montagerohr (7) am distalen Ende eine Prägung (36) zur Aufnahme eines senkrechten Halteelements (70), wie zum Beispiel eines Griffs, enthält.

11. Vorrichtung (1, 100) zur Befestigung von Wirbeln nach einem der vorhergehenden Ansprüche, **dadurch gekennzeichnet, dass** die beiden Halbschalen an ihrem distalen Ende durch einen Ring (40) aneinandergedrückt werden.

12. Vorrichtung (1, 100) zur Befestigung von Wirbeln nach Anspruch 11, **dadurch gekennzeichnet, dass** das Montagerohr (7) und der Ring (40) so angeordnet sind, dass sie eine steife Einheit bilden.

13. Vorrichtung (1, 100) zur Befestigung von Wirbeln nach einem der Ansprüche 1 bis 12, **dadurch gekennzeichnet, dass** die beiden Halbschalen (30,31) so angeordnet sind, dass sie, wenn sie aufeinander liegen, ein Führungsrohr bilden.

14. Vorrichtung (1, 100) zur Befestigung von Wirbeln nach Anspruch 13, **dadurch gekennzeichnet, dass** das durch die beiden Halbschalen gebildete Führungsrohr einen Innendurchmesser aufweist, der das Durchführen eines blockierenden Elements (3) des Verbindungselements bis auf das Knochenverankerungselement (2) ermöglicht.

15. Vorrichtung (1, 100) zur Befestigung von Wirbeln nach Anspruch 13 oder Anspruch 14, **dadurch gekennzeichnet, dass** das durch die beiden Halbschalen gebildete Führungsrohr einen Innendurchmesser aufweist, der das Durchführen der Hilfsmittel zum Einsetzen des blockierenden Elements ermöglicht.

16. Vorrichtung (1, 100) zur Befestigung von Wirbeln nach einem der Ansprüche 3 bis 15, **dadurch gekennzeichnet, dass** das blockierende Element (3) fest mit einem Trägerrohr (8) für das blockierende Element vormontiert wird, um eine blockierende Einheit zu bilden, wobei das blockierende Element (3) vom Trägerrohr (8) des blockierenden Elements abmontiert werden kann.

17. Vorrichtung (1, 100) zur Befestigung von Wirbeln nach Anspruch 16, **dadurch gekennzeichnet, dass** die blockierende Einheit steril verpackt ist.

18. Vorrichtung zur Befestigung von Wirbeln nach Anspruch 16 oder Anspruch 17, **dadurch gekennzeichnet, dass** die blockierende Einheit steril in der versiegelten Verpackung (1) verschlossen ist.

19. Vorrichtung (1, 100) zur Befestigung von Wirbeln nach einem der Ansprüche 16 bis 18, **dadurch gekennzeichnet, dass** das Trägerrohr (8) des blockierenden Elements so bemessen ist, dass es in das Montageohr (7) eingeschoben werden kann.

20. Vorrichtung (1, 100) zur Befestigung von Wirbeln nach Anspruch 19, **dadurch gekennzeichnet, dass** das Montagerohr (7) eine rohrförmige Innenwand aufweist, die mit mindestens einem Indexierungsrelief versehen ist, wobei das Trägerrohr des blockierenden Elements ein Indexierungsrelief aufweist, dessen Form das Indexierungsrelief des Montagerohrs (7) ergänzt.

21. Vorrichtung (1, 100) zur Befestigung von Wirbeln nach einem der Ansprüche 16 bis 20, **dadurch gekennzeichnet, dass** das Trägerrohr (8) des blockierenden Elements einen proximalen Teil aufweist, der mit einem Auflagerelief versehen ist, welches sich gegenüber der Verbindungsgrenzfläche des Knochenverankerungselements (2) befindet, wenn das Trägerrohr (8) des blockierenden Elements in das Montagerohr (7) eingeschoben wird, um ein Verbindungselement (6) auf die Verbindungsgrenzfläche des Knochenverankerungselements (2) zu drücken.

22. Vorrichtung (1, 100) zur Befestigung von Wirbeln nach einem der Ansprüche 16 bis 21, **dadurch gekennzeichnet, dass** es des Weiteren ein Schubelement (60) aufweist, das mit dem Trägerrohr (8) des blockierenden Elements zusammenwirkt, um das Trägerrohr (8) des blockierenden Elements in das Montageohr (7) zu schieben.

23. Vorrichtung (1, 100) zur Befestigung von Wirbeln nach einem der Ansprüche 16 bis 22, **dadurch gekennzeichnet, dass** das Montagerohr (7), wenn das Trägerrohr (8) des blockierenden Elements darin positioniert ist, ein Führungsrohr bildet.

24. Vorrichtung (1, 100) zur Befestigung von Wirbeln nach einem der Ansprüche 16 bis 23, **dadurch gekennzeichnet, dass** das Montageohr (7) und das Trägerrohr (8) des blockierenden Elements, wenn dieses darin positioniert ist, eine steife Einheit bilden.

25. Vorrichtung (1, 100) zur Befestigung von Wirbeln nach einem der Ansprüche 16 bis 24, **dadurch gekennzeichnet, dass** das Trägerrohr (8) des blockierenden Elements eine Innenform aufweist, die das Durchführen eines Schraubendrehers ermöglicht.

26. Vorrichtung (1, 100) zur Befestigung von Wirbeln nach einem der Ansprüche 16 bis 25, **dadurch gekennzeichnet, dass** das Trägerrohr (8) des blockierenden Elements aus Verbundmaterial, Polymer, Eisen- oder Nichteisenlegierung oder einer Kombination dieser Materialien gebildet wird.

27. Vorrichtung zur Befestigung von Wirbeln nach einem der vorhergehenden Ansprüche, **dadurch gekennzeichnet, dass** alle Teile der Vorrichtung zum einmaligen Gebrauch bestimmt sind.

28. Vorrichtung (1, 100) zur Befestigung von Wirbeln nach einem der vorhergehenden Ansprüche, **dadurch gekennzeichnet, dass** alle Teile der Vorrichtung in einer oder mehreren versiegelten Verpackungen (1,10) steril verschlossen sind.

## Claims

1. A spinal device (1, 100) for fixing vertebrae by minimally invasive surgery posterior or postero-lateral approach, with the device (1) comprising at least:
- a bone-anchoring element (2) of the pedicle or vertebral screw type, comprising a proximal portion (5) provided with an interface for fastening a connecting element (6) of the rod or plate type and a threaded distal portion (4)
**characterized in that** it comprises
- a mounting tube (7) integrally pre-assembled on the bone-anchoring element (2), with the mounting tube (7), consisting of two half-shells (30, 31) each have a proximal end engaged with the bone-anchoring element (2) and being integrally held together at the distal ends thereof and joined at their distal parts, being removable from the bone anchoring element (2), and
- a screwdriver bar (80) comprises an ancillary device going through the mounting tube (7) and having one end pre-assembled on the bone-anchoring element (2)
- a sterile packaging sealed package (1) of the bone-anchoring element (2) and the pre-assembled mounting tube (7) and screwdriver bar.

2. A spinal device (1, 100) for fixing vertebrae according to claim 1, **characterized in that** the mounting tube (7) comprises a longitudinal opening (32) opening into the proximal portion (5) of the bone-anchoring element (2) for the passage of the connecting element (6) .

3. A spinal device (1, 100) for fixing vertebrae according to claim 1 or claim 2, **characterized in that** the spinal device further comprises a locking element (3), with the inner diameter of the mounting tube (7) enabling the passage of the locking element (3) of the connecting element on the bone-anchoring element.

4. A spinal device (1, 100) for fixing vertebrae according to any one of claims 1 to 3, **characterized in that** the mounting tube (7) is provided with means making it possible to keep the ancillary device aligned with the bone-anchoring element (2).

5. A spinal device (1, 100) for fixing vertebrae according to claim 4, **characterized in that** the holding means comprise pins arranged on the inner face of the mounting tube (7).

6. A spinal device (1, 100) for fixing vertebrae according to claim 4 or claim 5, **characterized in that** the holding means comprise a holding fin (60') mounted on the end of the mounting tube (7) opposite the end supporting the bone-anchoring element (2).

7. A spinal device (1, 100) for fixing vertebrae according to claim 6, **characterized in that** the holding fin (60') is so arranged with the mounting tube (7) as to enable a rotational movement of the ancillary device inside the mounting tube (7).

8. A spinal device (1, 100) for fixing vertebrae according to any one of the preceding claims, **characterized in that** the mounting tube (7) is made of a composite material, a polymer, a ferrous or non-ferrous alloy, or a combination of these various materials.

9. A spinal device (1, 100) for fixing vertebrae according to any one of the preceding claims, **characterized in that** the mounting tube (7) is overmolded onto the bone-anchoring element.

10. A spinal device (1, 100) for fixing vertebrae according to any one of the preceding claims, **characterized in that** the mounting tube (7) has, on the distal end thereof, a recess (36) for positioning a perpendicular holding element (70) of the handle type.

11. A spinal device (1, 100) for fixing vertebrae according to any one of the preceding claims, **characterized in that** the two half-shells are held together at the distal ends thereof by means of a ring (40) .

12. A spinal device (1, 100) for fixing vertebrae according to claim 11, **characterized in that** the mounting tube (7) and the ring (40) are so arranged as to form a rigid assembly.

13. A spinal device (1, 100) for fixing vertebrae according to any one of claims 1 to 12, **characterized in that** the two half-shells (30, 31) are so arranged as to form, when they are integrally held together, a guide tube.

14. A spinal device (1, 100) for fixing vertebrae according to claim 13, **characterized in that** the guide tube formed by the two half-shells has an inner diameter enabling the passage of a locking element (3) of the connecting element on the bone-anchoring element (2).

15. A spinal device (1, 100) for fixing vertebrae according to claim 13 or claim 14, **characterized in that** the guide tube formed by the two half-shells has an inner diameter enabling the passage of ancillary devices providing the positioning of the locking element.

16. A spinal device (1, 100) for fixing vertebrae according to any one of claims 3 to 15, **characterized in that** the locking element (3) is integrally pre-assembled with a tube supporting the locking element (8) so as to form a locking assembly, with the locking element (3) being removable from the tube supporting the locking element (8).

17. A spinal device (1, 100) for fixing vertebrae according to claim 16, **characterized in that** the locking assembly is packed in a sterile packaging.

18. A spinal device (1, 100) for fixing vertebrae according to claim 16 or claim 17, **characterized in that** the locking assembly is packed in a sterile sealed packaging (1).

19. A spinal device (1, 100) for fixing vertebrae according to one of claims 16 to 18, **characterized in that** the tube supporting the locking element (8) has dimensions enabling the insertion thereof into the mounting pipe (7).

20. A spinal device (1, 100) for fixing vertebrae according to claim 19, **characterized in that** the mounting tube (7) has an inner tubular wall provided with at least one indexing pattern, with the tube supporting the locking element having a matching indexing pattern, the shape of which matches the indexing pattern of the mounting tube (7).

21. A spinal device (1, 100) for fixing vertebrae according to any one of claims 16 to 20, **characterized in that** tube supporting the locking element (8) has a proximal portion provided with a bearing pattern, located opposite the securing interface of the bone-anchoring element (2) when the tube supporting the locking element (8) is inserted into the mounting tube (7) for pressing a connecting element (6) onto the securing interface of the bone-anchoring element (2).

22. A spinal device (1, 100) for fixing vertebrae according to any one of claims 16 to 21, **characterized in that** it further comprises a pushing element (60) cooperating with the tube supporting the locking element (8) to insert the tube supporting the locking element (8) into the mounting tube (7).

23. A spinal device (1, 100) for fixing vertebrae according to any one of claims 16 to 22, **characterized in that** the mounting tube (7) forms a guide tube when the tube supporting the locking element (8) is positioned therein.

24. A spinal device (1, 100) for fixing vertebrae according to any one of claims 16 to 23, **characterized in that** the mounting tube (7) and the tube supporting the locking element (8) form a rigid assembly, when positioned therein.

25. A spinal device (1, 100) for fixing vertebrae according to any one of claims 16 to 24, **characterized in that** on the tube supporting the locking element (8) has an inner shape enabling the passage of a screwdriver.

26. A spinal device (1, 100) for fixing vertebrae according to any one of claims 16 to 25, **characterized in that** the tube supporting the locking element (8) is made of a composite material, a polymer, a ferrous or non-ferrous alloy, or still a combination of these various materials.

27. A spinal device (1, 100) for fixing vertebrae according to any one of the preceding claims, **characterized in that** all the parts of the device are disposable.

28. A spinal device (1, 100) for fixing vertebrae according to any one of the preceding claims, **characterized in that** all the parts of the device are packed in one or more sealed sterile packaging(s) (1, 10) .
